# EUROPEAN PATENT APPLICATION

(11) **EP 1 088 816 A2**
(43) Date of publication of application: **04.04.2001**
(21) Application number: 00204408.9
(22) Date of filing: 10.12.1994
(51) Int. Cl.: C07C 405/00, A61K 31/557

(54) **Use of certain prostaglandin analogues to treat glaucoma and ocular hypertension**

(30) Priority: 15.12.1993 US 167470; 15.12.1993 US 167747; 30.09.1994 US 316672
(62) Divisional of application: 94119571.1
(71) Applicant: ALCON LABORATORIES, INC., Fort Worth Texas 76134-2099 (US)
(72) Inventor: Sallee, Verney L., Burleson, Texas 76028 (US); Zinke, Paul W., Fort Worth, Texas 76133 (US); DeSantis, Louis, Jr., Fort Worth, Texas 76109 (US); Bishop, John E., Arlington, Texas 76016 (US)
(74) Representative: Hall, Marina

(57) **Abstract**

Certain D series prostaglandin analogues are effective in lowering intra ocular pressure and are useful in the treatment of glaucoma and ocular hypertension. Also disclosed are opthalmic, pharmaceutical compositions comprising such prostaglandin analogues and their use.

## Description

### Background of the Invention

The present invention relates to the use of certain prostaglandins and prostaglandin analogues for the treatment or glaucoma and ocular hypertension. As used herein, the terms "prostagiandin" and "PG" shall refer to prostagiandins and derivatives and analogues thereof, except as otherwise indicated by context.

Naturally-occurring prostaglandins are known to lower intraocular pressure (IOP) after topical ocular instillation, but generally cause inflammation, as well as surface irritation characterized by conjunctival hyperemia and edema. Many synthetic prostaglandins have been observed to lower intraocular pressure. but such compounds also produce the aforementioned side effects. Various attempts have been made to overcome the ocular side effects associated with prostaglandins. Stjernschantz et al. (EP 364 417 B1) have synthesized derivatives or analogues of naturally-occurring prostaglandins in order to design out selectively the undesired side effects while maintaining the IOP-lowering effect. Others, including Ueno et al. (EP 330 511 A2) and Wheeler (EP 435 682 A2) have tried complexing prostaglandins with various cyclodextrins.

EP-B-0 656 889 has a priority date of 31st August 1992 and was published on 17th March 1994 and is relevant only under Article 54(3) and (4) EPC. EP-B-0 656 889 discloses pharmaceutical preparations comprising esters or amides of 9-chloroprostaglandins of formula (I) 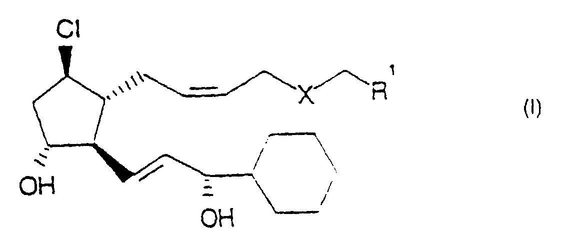 in which
- X: represents oxygen or CH₂, and
- R¹: represents COOR² wherein R² is an optionally substituted C₁-C₁₀alkyl, C₃-C₁₀-cycloalkyl, C₆-C₁₀aryl or C₆-C₁₀ar(C₁-C₄)alkyl radical.
or CONHR³ wherein R³ is an optionally substituted C₁-C₁₀alkyl radical, for treating raised intraocular pressure, and to pharmaceutical preparations comprising 9-chloroprostaglandin esters or amides of the general formula (I).

WO92/08697 relates to 9-halogen-11β-hydroxy-prostane derivatives of formula I 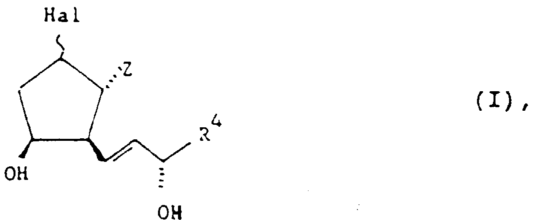 in which Z means the radicals

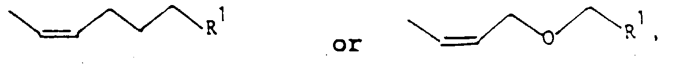

Hal means an α- or β-position chlorine or tluorine atom, R¹ means the radical 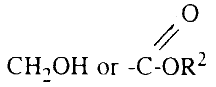 with R² meaning a hydrogen atom, an alkyl, cycloalky, aryl or heterocyclic radical or R¹ means the radical 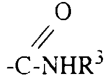 with R³ meaning an acid radical or radical R² and R⁴ means a cycloalkyl group, and if R² has the meaning of a hydrogen atom. its salts with physiologically compatible bases and its cyclodextrin clathrates,
which are especially suitable for oral administration.

WO86/04504 relates to a pharmaceutical preparation which contain as active compound cyclodextrine clathrate of prostaglandin having the general formula (I) 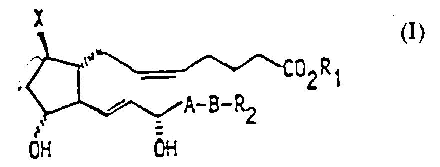 wherein R₁ is a hydrogen atom or a straight or branched chain alkyl radical having up to 10 atoms of carbon. R₂ is an alkyl, cycloalkyl, or optionally substituted phenyl group, A and B form together a direct bond or A is a straight or branched chain alkyl group having up to 10 atoms of carbon and B is an oxygen atom, a direct bond or -C = C bond and X is a chlorine or fluorine atom. The clathrate compounds according to WO86/04504 exhibit strong inhibiting activity on gastric acid secretion and protect the mucosa of the stomach against various deleterious influences, as well as against anti-inflammatory substances. Several of the clathrates according to this reference are excellently suited for the treatment of allergic and vasomotor rhinitis, and several of the clathrates are furthermore suitable, upon enteral or parenteral administration, for inducing menstruation or interrupting pregnancy. They are also suitable for synchronizing the sexual cycle of female mammals such as rabbits, cattle, horses and pigs and for cervix dilation as a preparation for diagnostic or therapeutic interventions. The clathrates of W086/04504 can be utilized in liquid or solid galenic formulations, and the formulations can be administered enterally, parenterally, vaginally or rectally.

DE-A-4 229 053 provides esters and amides of 9-chloro-prostaglandins which have a similar spectrum of activity to the corresponding natural prostaglandins, to promote blood circulation in the skin and can be used to heal skin lesions.

EP-A-0 299 914 relates to a 9-halogen-(Z) prostaglandin derivative of formula I 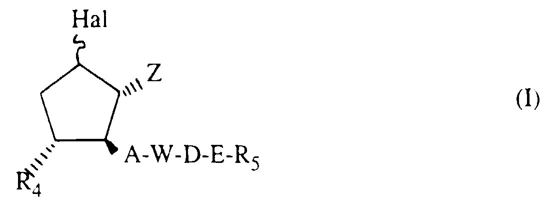 in which
Z represents the radicals 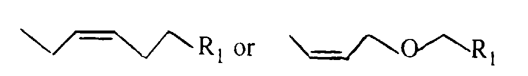 Hal represents a chlorine or fluorine atom in the alpha or beta position.
R₁ represents the radical CH₂OH or 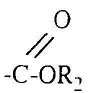 with R₂ meaning a hydrogen atom, an alkyl, cycloalkyl, aryl or heterocyclic radical or R₁ represents the radical 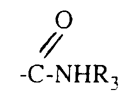 with R₃ meaning an acid radical or the radical R₂ and
A represents a -CH₂-CH₂-, a trans-CH=CH or -C≡C group,
W represents a free or a functionally modified hydroxymethylene group or a free or functionally modified 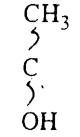 group, and the respective OH groups can be in the alpha or beta position,
D and E together represent a direct bond or
D represents a straight-chain alkylene group with 1-10 C atoms, a branched-chain alkylene group with 2-10 C atoms or an annular alkylene group with 3-10 C atoms, which optionally can be substituted by fluorine atoms, and
E represents an oxygen or sulfur atom, a direct bond. a-C≡C bond or a -CR₆=CR₇ group, and R₆ and R₇ are different and mean a hydrogen atom, a chlorine atom or a C₁-C₄ alkyl group,
R₄ represents a free or functionally modified hydroxy group,
R₅ means a hydrogen atom, an alkyl, a halogen-substituted alkyl, a cycloalkyl, an optionally substituted aryl or a heterocyclic group, and if R₂ means a hydrogen atom, its salts with physiologically compatible bases or its cyclodextrin clathrates

The 9-halogen-(Z) prostaglandins of EP-A-0 299 914 are especially suitable for oral application.

WO86/05488 relates to 9-halo-15-cycloalkyl prostaglandin derivatives of Formula I 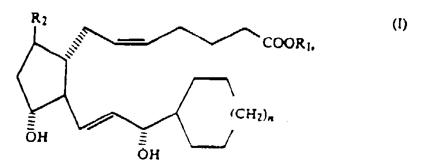 wherein
R₁ is hydrogen or methyl.
R₂ is fluorine or chlorine.
n is 0 or 1,
and, if R₁ is hydrogen, the salts thereof with physiologically compatible bases or the cyclodextrin clathrates thereof.

The compounds of WO86/05488 inhibit gastric acid secretion, exhibit a cytoprotective and ulcer-healing activity, exert a regulatory effect in cardiac arrhvthmias and inhibit platelet aggregation.

WO92/02495 discloses prostaglandins derived from cyclopentanes having a fluorine atom or a hydroxy group in the 9-position of the prostane. The compounds are suitable for the treatment of diseases of the cardiovascular system, the stomach, the pancreas. the liver and the kidneys. They have a blood pressure-reducing and bronchodilatory action and are suitable for the inhibition of thrombocyte activation.

### Summary of the Invention

It has now been unexpectedly discovered that certain D series prostaglandin analogues are significantly more effective in lowering IOP than other known prostaglandins. In particular. the prostaglandin analogues of the present invention have unexpectedly been found to lower IOP to a greater degree than other known PGs without significant inflammation or associated side effects which often accompany topical ocular administration of D series prostaglandins.

### Detailed Description of the Invention

The prostaglandins which are useful in the compositions of the present invention have the general formulae (I) and (II): 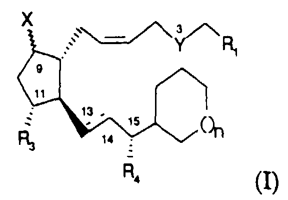 wherein:
- R₁ =: CO₂R₂, wherein R₂ = H, a cationic salt moiety, or an ophthalmically acceptable ammonium moiety; or R₁ may also represent an ophthalmically acceptable ester moiety;
- X =: halogen, particularly Cl or F, in either configuration;
- Y =: CH₂ or O;
- --- =: single or *trans* double bond;
- R₃, R₄: can be the same or different, and are selected from: free or functionally modified hydroxy groups; and
- n =: 0 or 1. 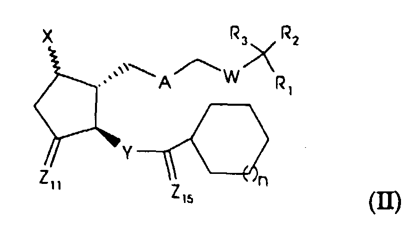
wherein:
- R₁ =: CH₂R, CO₂R₄;
- R =: OH or functionally modified hydroxy group;
- R₂ and R₃: can be the same or different and are selected from: H and CH₃;
- R₄ =: H, a cationic salt moiety, an ophthalmically acceptable ammonium moiety, substituted or unsubstituted alkyl, cycloalkyl, (cycloalkyl)alkyl, aryl, arylalkyl, heteroaryl, or (heteroaryl)alkyl,wherein substituents include alkyl, halo, a free or functionally modified hydroxy group, or a free or functionally modified thiol;
- W =: CH₂, O, S(O)ₘ wherein m = 0, 1, 2;
- A =: CH₂CH₂, *cis* or *trans* CH=CH, or C≡C;
- X =: Cl, F or R in either configuration, or H;
- Z₁₁: may be selected from O (i.e. a carbonyl), H and R in either configuration, or H and H;
- Z₁₅: may be selected from O (i.e., a carbonyl) H and R in either configuration or H and H;
- Y =: CH₂CH₂ or *trans* CH=CH, or C≡C; and
- n =: 0 or 1.

The parenthesis symbol () in the ring structure of formulae (I) and (II) represents the fact that the ring may be 5-membered or 6-membered.

As used in this specification, the term "ophthalmically acceptable ester moiety" refers to an ester moiety which is non-toxic and does not cause undue irritation to the ocular tissues. For compounds of formula (I), examples of ophthalmically acceptable esters include, but are not limited to: R₂ = substituted or unsubstituted alkyl, cycloalkyl, (cycloalkyl)alkyl, aryl, arylalkyl, heteroaryl, or (heteroaryl)alkyl, wherein substituents include alkyl, halo, a free or functionally modified hydroxy group, or a free or functionally modified thiol. As used in this specification, the term "heteroaryl" refers to a monocyclic ring system of 5 or 6 atoms composed of C, N, O, and/or S, such as furan, thiophene, pyrrole, pyrazole, imidazole, triazole, tetrazole, oxazole, isothiazole, thiazole, thiadiazole, pyridine, pyrimidine, pyradazine and pyrazine. The term "functionally modified hydroxy groups" refers to either etherified hydroxy groups or acylated (esterified) hydroxy groups. Similarly, the term "functionally modified thiol" refers to an alkylated thiol.

Preferred compounds of formula (I) include those wherein: R₁ = CO₂R₂; R₂ = H, methyl, ethyl, *n*-propyl, isopropyl, *t*-butyl, or benzyl; X = Cl in the β (*R*) configuration; Y = O or CH₂; R₃ and R₄ = OH; and n = 1. The most preferred compounds of formula (I) are those wherein: R₁ = CO₂R₂; R₂ = H, methyl, ethyl, isopropyl, or t-butyl; X = Cl in the β (*R*) configuration; Y = O; R₃ and R₄ = OH; and n = 1.

Preferred compounds of formula (II) include those wherein: W = CH₂ or O; A = *cis* CH=CH; X = Cl or H; Z₁₁ = R and H in either configuration or H and H; Y = CH₂CH₂, or *trans* CH=CH; and n = 1. The most preferred compounds of formula (II) are those wherein: R₂, R₃ = H; X = Cl in β (*R*) configuration or H; Z₁₁ = H and R in either configuration; Z₁₅ = H and R in either configuration; R₁=CO₂R₄; and R₄=H, a cationic salt moiety, or substituted or unsubstituted C₁-C₁₀ alkyl.

Some of the compounds of formula (II) are novel. The novel compounds of formula (II) are those wherein: R₁ = CH₂R, CO₂R₄; R = OH or a functionally modified hydroxy group; R₂ and R₃ can be the same or different and are selected from: H and CH₃; R₄ = H, a cationic salt moiety, substituted or unsubstituted alkyl, cycloalkyl, (cydoalkyl)alkyl, aryl, arylalkyl, heteroaryl, or (heteroaryl)alkyl, wherein substituents include alkyl, halo, a free or functionally modified hydroxy group, or a free or functionally modified thiol; W = O, S(O)ₘ, wherein m = 0, 1, 2; A = CH₂CH₂, *cis* or *trans* CH=CH, or C≡C; X = H; Z₁₁ and Z₁₅ may be the same or different and may be selected from O (i.e., a carbonyl), H and R in either configuration, or H and H; Y = CH₂CH₂ or *trans* CH=CH, or C≡C; and n = 0 or 1.

The preferred novel compounds of formula (II) are those wherein: R₁ = CO₂R₄; R₂ and R₃ = H; R₄=H, a cationic salt moiety, or a substituted or unsubstituted C₁-C₁₀ alkyl; W = O, A = *cis* CH=CH; Z₁₁ = R and H in either configuration; Y = CH₂CH₂, or *trans* CH=CH; Z₁₅ = R and H in either configuration; and n = 0 or 1.

The following Table 1 contains examples of some preferred compounds of the present invention.

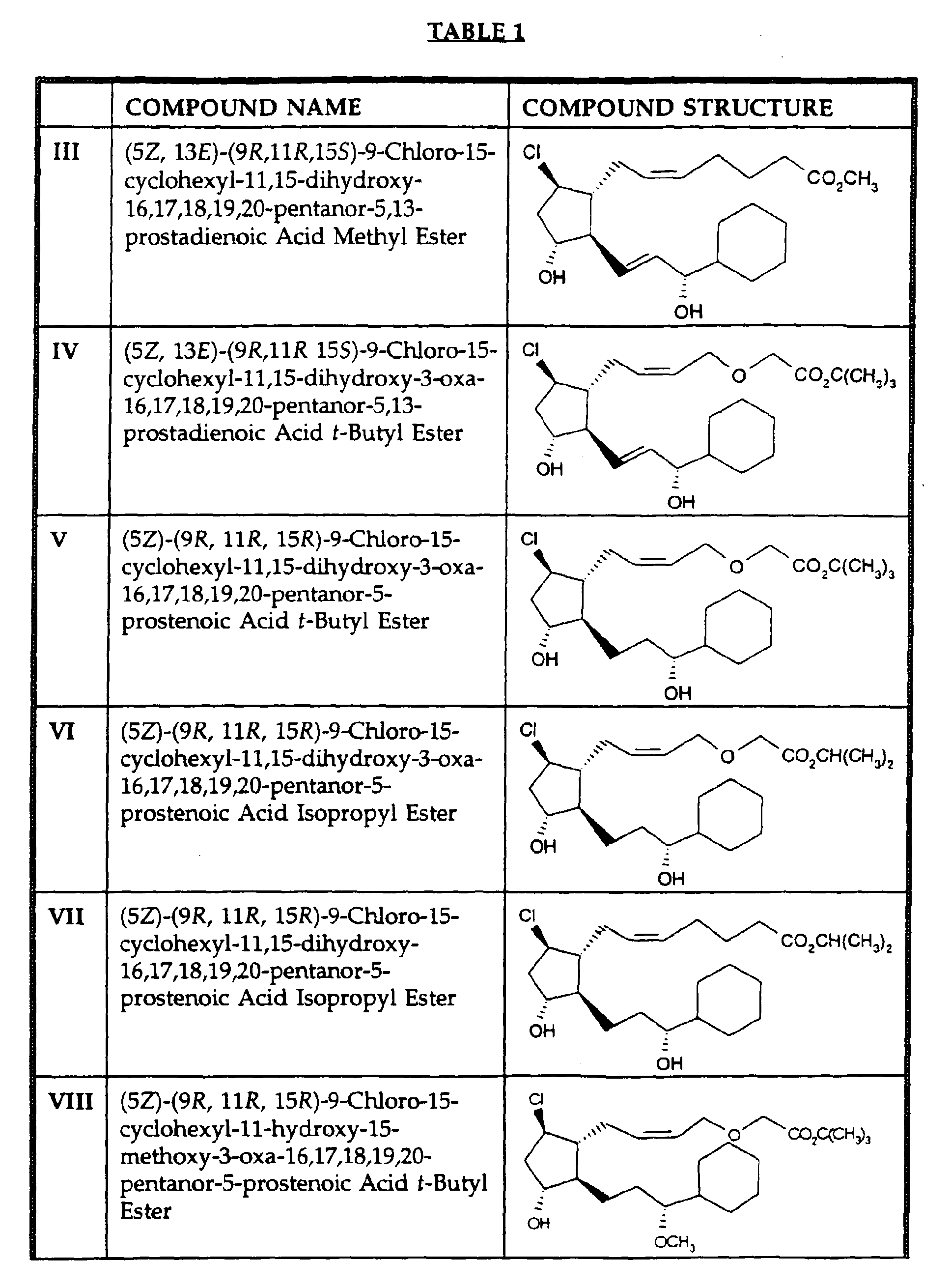

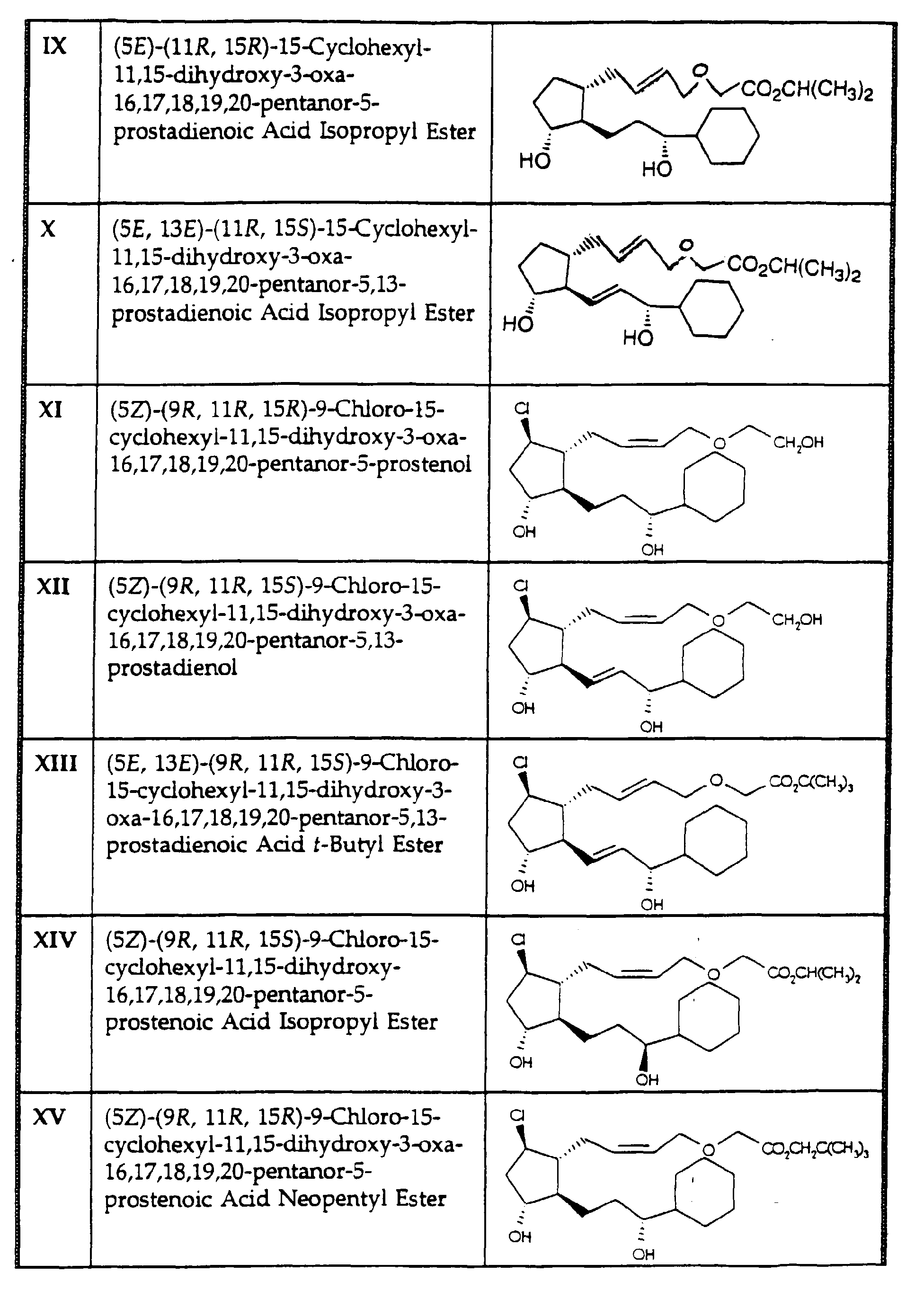

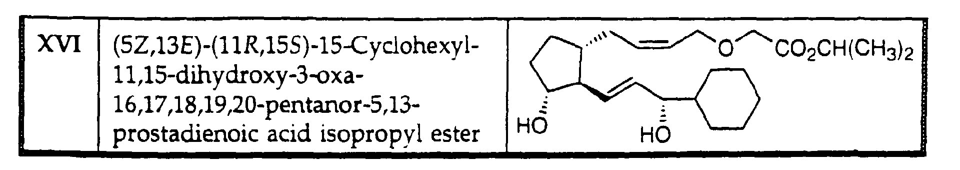

Some of the above-mentioned prostaglandins are disclosed in US 5,004,752 (Raduechel et al.) and EP 299 914 (Buchmann et al.). To the extent that US 5,004,752 and EP 299 914 teach the preparation of the prostaglandins of the present invention, these patents are hereby incorporated by reference herein. In addition, the syntheses of some of the prostaglandins of Table 1, above, are detailed below in Examples 1-12.

In the following Examples 1-12, the following standard abbreviations are used: g = grams (mg = milligrams); mol = moles (mmol = millimoles); mol% = mole percent; ml = milliliters; mm Hg = millimeters of mercury; mp = melting point; bp = boiling point; h = hours; and min = minutes. In addition, "NMR" refers to nuclear magnetic resonance spectroscopy and "CI MS" refers to chemical ionization mass spectrometry.

### EXAMPLE 1: SYNTHESIS OF COMPOUND V

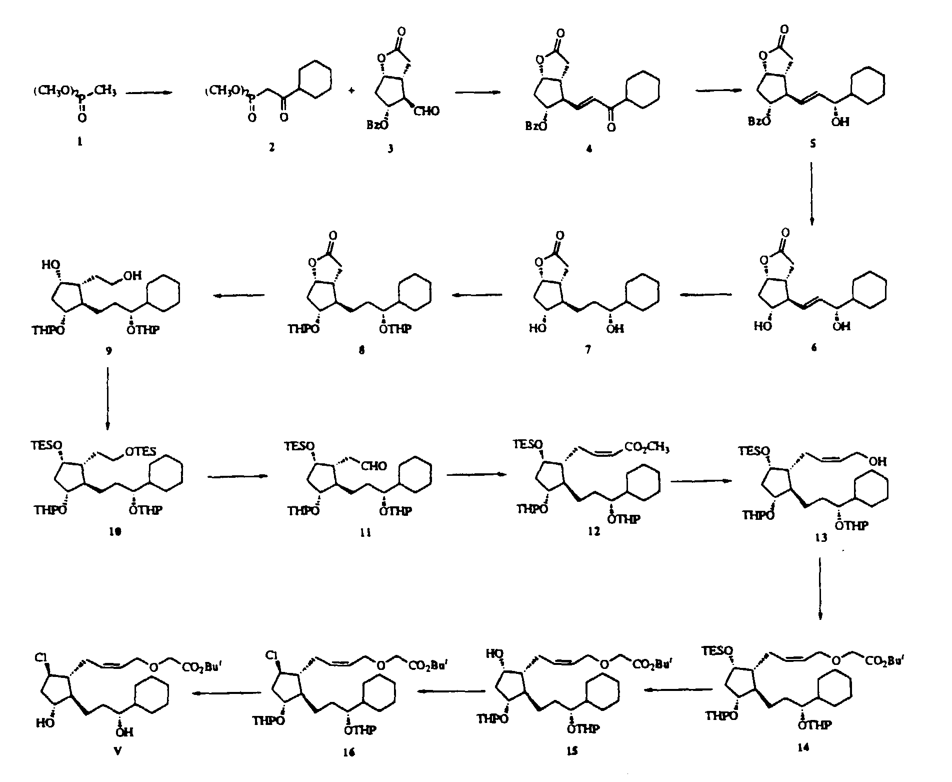

### A: Dimethyl (2-cyclohexyl-2-oxo)ethylphosphonate (2):

A solution of dimethyl methylphosphonate (100 g, 0.8 mol) in 1.0 L of anhydrous THF was cooled to -70°C and n-BuLi (2.5 *M* in hexanes, 320 ml, 0.8 mol) was added dropwise such that the temperature remained below -60°C. The mixture was stirred for 10 min at -70°C and then methyl cyclohexanecarboxylate (57.3 ml, 0.4 mol) was added dropwise, via syringe, over a period of 15 min. The resulting mixture was then stirred for 14 h at room temperature. The reaction was quenched by first cooling to 0°C followed by the addition of 2 *M* HCl until the aqueous layer was at pH 2. The layers were separated and the aqueous layer was extracted with 2 X 200 m 1 of CH₂Cl₂. The organic layers were combined and washed sequentially with 200 m 1 each of water and brine and then dried (MgSO₄). Filtration and solvent removal gave a yellow oil which was distilled under vacuum to afford 67.3 g (72%) of 2 as a clear colorless liquid: bp=100-115°C (0.01 mmHg); ¹H NMR (CDCl₃) δ 3.74 (d, *J* = 12.0 Hz, 6H), 3.08 (d, *J* = 22 Hz, 2H), 2.55 (m. 1H), 1.95- 1.60 (m, 5H), 1.40 - 1.15 (m, 5H).

### B: (3aR. 4R, 5R, 6aS)-5-(Benzoyloxy)-4-[(E)-3-cyclohexyl-3-oxo-1-propenyl]-hexahydro-2H-cyclopenta[b]furan-2-one (4):

A solution of anhydrous THF (1.4 L), LiCl (11.7 g, 0.28 mol) and the phosphonate **2** (67.0 g, 0.28 mol) was cooled to 0°C and triethylamine (39.2 ml, 0.28 mol) was added dropwise. A solution of the aldehyde **3** (68.5 g, 0.25 mol) in dry CH₂Cl₂ (320 ml) was added dropwise to the cold suspension and the resulting mixture was stirred at 0°C for 3 h. The reaction mixture was then poured into 500 ml of 2 *M* HCl, and layers were separated. The aqueous layer was extracted with 500 ml of CH₂Cl₂. The combined organic layers were washed with 100 ml each of water and brine followed by drying over MgSO₄. Filtration and solvent removal gave a yellow solid which was recrystallized from EtOAc to afford 85.8 g (89%) of **4** as a white solid: mp 151-153°C; ¹H NMR (CDCl₃) δ 8.01 (d, *J* = 2.0 Hz, 2H), 7.65 - 7.40 (m, 3H), 6.70 (dd, *J* = 12, 6 Hz, 1H), 6.35 (d, *J* = 12 Hz, 1H), 5.32 (m, 1H), 5.15 (m, 1H), 2.93 (m, 3H), 2.72 - 2.25 (m, 4H), 1.85 - 1.56 (m, 6H), 1.40-1.15 (m, 5H).

### C: (3aR, 4R, 5R, 6aS)-5-(Benzoyloxy)-4-[(E)-(3S)-3-cyclohexyl-3-hydroxy-1-propenyl]-hexahydro-2H-cyclopenta[b]furan-2-one (5):

A solution of CeCl₃•7H₂O (19.5 g, 52.3 mmol) and enone **4** (20.0 g, 52.3 mmol) in 150 ml of CH₃OH and 70 ml of CH₂Cl₂ was prepared. NaBH₄ (1.92 g, 52.3 mmol) was added in small portions over a period of 5 min. The resulting mixture was stirred at ambient temperature for 45 min and then was poured into a separatory funnel containing 100 ml each of 25% (v/v) aqueous acetic acid and CH₂Cl₂. The layers were separated and the aqueous layer was extracted with 3 X 50 ml of CH₂Cl₂. The combined organic layers were washed with sat. NaHCO₃ (50 ml), and brine (50 ml), and then dried (MgSO₄). Upon solvent removal, 23.7 g of a colorless oil containing nearly equal amounts of the two diastereomeric allvl alcohols was obtained. The diastereomers were separated by HPLC (40% EtOAc/hexane), affording **5** (9.34 g (46%), the less polar component) as a white solid. ¹H NMR (CDCl₃) δ 8.01 (d, *J* = 8 Hz, 2H), 7.62 - 7.28 (m, 3H), 5.61 (m, 2H), 5.25 (m, 1H), 5.08 (m, 1H), 3.85 (m, 1H), 2.95 - 2.45 (m, 5H), 2.30 (m, 2H), 1.95-1.55 (m, 6H), 1.50 - 0.80 (m, 5H).

### D: (3aR, 4R, 5R, 6aS)-4-[(3R)-3Cyclohexyl-3-hydroxypropyl]-hexahydro-5- hydroxy-2H-cyclopenta[b]furan-2-one (7):

A solution of the allyl alcohol **5** (10.0 g, 26.0 mmol) in warm methanol (100 m 1) was cooled to ambient temperature. Anhydrous K₂CO₃ (3.6 g, 26.0 mmol) was added and the resulting mixture was stirred at ambient temperature for 3 h. The reaction mixture was concentrated and the residue was partitioned between 100 ml each of EtOAc and 1 *M* HCl. The layers were separated and the aqueous phase was extracted with 3 X 50 ml of EtOAc. The combined organic layers were washed with 50 m1 of water, 2 X 50 ml of sat. NaHCO₃, 50 ml of brine, and dried over MgSO₄. Filtration and evaporation gave the diol **6** (9.8 g, 92% yield, R =0.26, 100% EtOAc), which was used in the subsequent reaction without further purification

The crude diol 6 (9.8 g, 26 mmol) was dissolved in 50 ml of EtOAc and a catalytic amount (0.1 g) of 5% Pd/C was added. This mixture was hydrogenated at 30-40 psi in a Parr hydrogenation apparatus for 3 h and then filtered through a short pad of Celite. The filtrate was concentrated and the crude yellow oil was purified by passage through a short column of silica (R_{*f*} =0.26, EtOAc) to afford 7 (5.06 g, 70% yield from 5) as a colorless, viscous oil which solidified upon standing. ¹H NMR (CDCl₃) δ 4.95 (m, 1H), 4.05 (m, 1H), 3.35 (m, 1H), 2.80 (m, 1H), 2.58 (m, 2H), 2.30 (m, 1H), 2.00 (m, 14H).

### E: (3aR, 4R, 5R, 6aS)-4-[(3R)-3-Cyclohexyl- 3-(tetrahydropyran-2-yloxy)propyl]-hexahydro-5-(tetrahydropyran-2-yloxy)-2H-cyclopenta[b]furan-2-one (8):

A solution of the diol 7 (6.0 g, 21.2 mmol) and dihydropyran (7.80 ml, 84.8 mmol) in CH₂Cl₂ (100 ml) was cooled to 0°C. A catalytic amount of ρ-TsOH (0.05 g, 0.26 mmol) was added and the mixture was stirred for 30 min at 0°C. The reaction was then quenched by adding sat. aqueous NaHCO₃ (10 ml). The layers were separated and the aqueous phase was extracted with 2 X 25 ml of CH₂Cl₂. The combined organic layers were dried over anhydrous K₂CO₃, filtered and concentrated to afford a colorless oil which was purified by passage through a short column of silica (R_{*f*} =0.46, 1:1 EtOAc/hexanes). The bis-THP ether 8 (8.59 g, 89% yield) was isolated as a colorless oil which solidified upon standing. ¹H NMR (CDCl₃) δ (characteristic peaks only) 5.00 (m, 1H), 4.75 - 4.45 (m, 2H), 3.85 (m, 2H), 3.60 - 3.30 (m, 4H).

### F: (9S, 11R, 15R)-11,15-Bis-(tetrahydropyran-2-yloxy)-15-cyclohexyl-23,4,5,6,16,17,18,19,20-decanor-9-(triethylsilyloxy)prostanol Triethylsilyl Ether (10):

A suspension of lithium aluminum hydride (1.43 g, 38.0 mmol) in 50 ml of anhydrous THF was cooled to 0°C and a solution of the lactone **8** (8.59 g, 19.0 mmol) in THF (100 ml) was added dropwise. The resulting mixture was stirred at 0°C for 3 h, after which 1.5 ml of H₂O, 1.5 ml of 15% NaOH and 4.5 ml of H₂O were sequentially added. After warming to ambient temperature, 100 ml of EtOAc was added and the solids were filtered off. The filter cake was washed thoroughly with 3 X 50 ml of EtOAc and the filtrates were dried by passage through a short pad of anhydrous MgSO₄. Evaporation afforded 9 (9.02 g) as a colorless oil which was used in the subsequent step without further purification (R_{*f*} =0.31, 80:20 EtOAc/hexanes).

A mixture of the crude diol 9 (9.02 g, 19.0 mmol), triethylsilyl chloride (9.65 ml, 57.0 mmol), dimethylaminopyridine (0.41 g, 3.42 mmol), triethylamine (16.0 ml 114 mmol) and anhydrous *N,N*-dimethylformamide (50 ml) was stirred at ambient temperature for 14 h under N₂. The reaction mixture was then diluted with 250 ml of CH₂Cl₂ and the solution was washed with 3 X 50 ml of H₂O. The combined water washes were extracted with 2 X 50 ml of CH₂Cl₂. The organic layers were combined, dried (MgSO₄), filtered and concentrated to afford a yellow oil which was chromatographed on silica (R_{*f*} =0.4, 1:9 EtOAc/hexanes). Pure **10** (11.23 g, 86% yield from 8) was obtained as a slightly yellow oil. ¹H NMR (CDCl₃) δ (characteristic peaks only) 4.62 (m, 2H), 4.15 - 3.25 (broad m, 7H), 2.30 - 1.15 (broad m, 18H), 0.95 (broad t, 18H), 0.65 (broad q, 12H).

### G: (9S, 11R, 15R)-11,15-Bis-(tetrahydropyran-2-yloxy)-15-cyclohexyl-2,3,4,5,6,16,17,18,19,20-decanor-9-(triethylsilyloxy)prostanal (11):

A solution of oxalyl chloride (0.51 ml, 0.57 mmol) in anhydrous CH₂Cl₂ (15 ml) was cooled to -78°C under N₂. A solution of anhydrous DMSO (0.81 ml, 11.4 mmol) in CH₂Cl₂ (2.0 ml) was then added dropwise. After 2 min, a solution of **10** (2.6 g, 3.8 mmol) in 8 ml of dry CH₂Cl₂ was introduced dropwise via syringe over a period of 2 min. The resulting mixture was stirred at -78°C for 2 h at which time triethylamine (2.7 ml, 19.0 mmol) was added. The reaction was stirred for 15 min and then allowed to warm to ambient temperature. The mixture was partitioned between 100 ml of EtOAc and 10 ml of H₂O and the organic layer was washed with an additional 10 ml of H₂O, 10 ml of brine and dried (MgSO₄). Solvent removal gave a yellow oil which was subjected to chromatography on silica gel (R_{f} 0.2, 10% EtOAc/hexanes) to afford **11** (1.4 g, 65% yield) and some starting material (0.83 g). ¹H NMR (CDCl₃) δ 9.80 (broad s, 1H), 4.62 (m, 2H), 4.20 (m, 1H), 3.85 - 3.60 (m, 3H), 3.40 (m, 3H), 2.80 (m, 1H), 2.45-2.05 (m, 4H), 1.95 - 1.10 (broad m, 27H), 0.95 (broad t, 9H), 0.55 (broad q, 6H).

### H: (5Z)-9S, 11R, 15R)-11,15-Bis-(tetrahydropyran-2-yloxy)-15-cyclohexyl-2,3,4,16,17,18,19,20-octanor-9-(triethylsilyloxy)-5-prostenoic Acid Methyl Ester (12):

A solution of 18-crown-6 (8.50 g, 32.1 mmol) and bis(2,2,2-trifluoroethyl) (methoxycarbonylmethyl)phosphonate (3.72 g, 11.7 mmol) in 110 ml of THF was cooled to -78°C. KHMDS (0.5 *M* in toluene, 23.4 ml, 11.7 mmol) was added to the above mixture and the solution was stirred for 15 min. Aldehyde **11** (6.11 g, 10.7 mmol) in 5.0 ml of THF was added dropwise over a period of 15 min. The reaction was stirred at -78°C for 2 h, then warmed up to 0°C and stirred at that temperature for 2 more hours. The reaction was quenched by adding 50 ml of saturated aqueous NH₄Cl and the mixture was allowed to warm to room temperature. The layers were separated and the aqueous layer was extracted with 2 X 50 ml of EtOAc. The combined organic layers were washed with 2 X 50 ml of brine and dried (K₂CO₃). Filtration and solvent removal gave a crude yellow oil which was purified by passage through a short plug of silica to afford a mixture of **12** and its E isomer (9:1 ratio, 6.28 g, 95% yield). The isomers were separated by chromatography on silica gel (R_{f} =0.56, and 0.47, for the major and minor isomers respectively, 40% Et₂O/hexane); 4.57 g of pure **12** and 0.97 g of a 1:1 E/Z mixture were isolated. ¹H NMR (CDCl₃) 6 6.35 (m, 1H), 5.78 (broad d, J = 12.0 Hz, 1H), 4.65 (m, 2H), 4.28 (m, 1H), 3.90 (m, 2H), 3.70 (s, 3H), 3.55-3.30 (m, 3H), 2.80 (m, 2H), 2.35-2.05 (m, 1H), 2.00-1.10 (broad m, 30H), 0.95 (broad t, 9H), 0.60 (broad q, 6H).

### I: (5Z)-(9S, 11R, 15R)-11,15-Bis-(tetrahydropyran-2-yloxy)-15-cyclohexyl-2,3,4,16,17,18,19,20-octanor-9-(triethylsilyloxy)-5-prosten-1-ol (13):

A solution of **12** (2.0 g, 3.22 mmol) in 20 ml of anhydrous THF was cooled to 0°C under N₂. A solution of diisobutylaluminum hydride (1.5 *M* in toluene, 6.5 ml, 9.66 mmol) was added dropwise and the resulting mixture was stirred at 0°C for 2 h. The reaction was then quenched by careful addition of CH₃OH (5 ml), allowed to warm up to ambient temperature, and diluted with 50 ml of THF. The resulting cloudy solution was treated with 50 ml of a saturated aqueous solution of sodium potassium tartrate and the biphasic mixture was stirred for 1 h. The layers were then separated and the aqueous layer was extracted with 2 X 50 ml of THF. The organic extracts were combined, washed with brine (50 ml), and dried (MgSO₄). Filtration and solvent removal gave a pale yellow oil which was purified by chromatography on silica gel (R_{*f*} =0.26, 4:6 Et₂O/hexane) to yield **13** (1.95 g, 95% yield) as a colorless oil. This compound was used immediately in the subsequent reaction. ¹H NMR (CDCl₃) δ 5.65 (m, 2H), 4.65 (m, 2H), 4.30-3.25 (broad m, 5H), 2.40-2.05 (broad m, 4H), 2.00-1.10 (broad m, 32H), 1.00 (broad t, 9H), 0.60 (broad q, 6H).

### J: (5Z)-(9S, 11R, 15R)-11,15-Bis-(tetrahydropyran-2-yloxy)-l5-cyclohexyl-9-hydroxy-3-oxa-16,17,18,19,20-pentanor-5-prostenoic Acid t-Butyl Ester (15):

A mixture of **13** (1.95 g, 3.28 mmol), *t*-butyl bromoacetate (5.11 g, 26.24 mmol), tetrabutylammonium hydrogen sulfate (0.8 g, 2.35 mmol), toluene (45 ml) and 25% (w/w) aqueous NaOH (30 ml) was stirred vigorously at room temperature for 18 h. The layers were separated and the aqueous layer was extracted with 2 X 25 ml of EtOAc. The combined organic extracts were washed with brine (15 ml), dried (MgSO₄), and concentrated. The crude product was purified by chromatography on silica gel (R_{*f*}=0.56, 20% EtOAc/hexane) to yield 2.19 g of **14** (contaminated with some *t*-butyl bromoacetate) and 0.48 g of the starting allyl alcohol **13**. The allyl ether **14** thus obtained was used in the desilylation reaction without further purification.

The silyl ether **14** (0.5 g) obtained above was dissolved in 3.0 ml of DMSO and to it was added 2.2 ml of tetrabutylammonium fluoride (1.0 *M* in THF, 2.2 mmol). The mixture was stirred at ambient temperature for 30 min and then partitioned between 50 ml EtOAc and 10 ml of brine. The aqueous layer was extracted with 2 X 10 ml of EtOAc and the combined organic extracts were dried over MgSO₄. Evaporation and chromatography on silica gel (R_{*f*} =0.44, 50% EtOAc/hexane) afforded 0.28 g of **15** as a colorless oil. ¹H NMR (CDCl₃) δ 5.65 (m, 2H), 4.62 (m, 2H), 4.16 (m,. 1H), 4.10-3.75 (m, 3H), 3.95 (s, 2H), 3.45 (m, 2H), 2.50-0.90 (broad m, 35H), 1.46 (s, 9H); High Resolution CI MS *m/z* (CI) calcd. for C₃₄H₅₉O₈ (MH⁺) 595.4209, found 595.4208.

### K: (5Z)-(9R, 11R, 15R)-9-Chloro-15-cyclohexyl-11,15-dihydroxy-3-oxa-16,17,18,19,20-pentanor-5-prostenoic Acid t-Butyl Ester (V):

The hydroxyester **15** (0.28 g, 0.47 mmol) was dissolved in 4.0 ml of a stock solution containing 48.0 ml of CH₃CN, 0.79 ml of pyridine, and 0.97 ml of CCl₄. Triphenylphosphine (0.18 g, 0.70 mmol) was added and the resulting mixture was stirred at ambient temperature for 17 h. The reaction mixture was treated with 10 ml of a 1:1 solution of Et₂O/hexanes and the precipitate formed was filtered off. The filtrate was concentrated and purified by chromatography (silica gel, R_{*f*} =0.47, 40:60 Et₂O/hexanes) to yield pure **16** (90 mg, 34%) as a colorless oil.

A solution of **16** (80 mg, 0.13 mmol) in 7.0 ml of 65% (v/v) aqueous acetic acid was heated to 65-70°C for 45 min. The reaction mixture was cooled to room temperature and concentrated. The resulting residue was redissolved in anhydrous EtOH and the solvent was again evaporated. The residue thus obtained was purified by chromatography on silica gel (R =0.4, 60:40 EtOAc/hexanes) to yield 60 mg (100%) of V as a colorless, viscous oil. ¹H NMR (CDCl₃) δ 5.69 (m, 2H), 4.32 - 3.85 (m, 5H), 3.38 (m, 1H), 2.50 - 1.95 (m, 5H), 1.95-0.80 (broad m, 29H) 1.43 (s, 9H); ¹³C NMR (CDCl₃) δ 169.9, 131.7, 126.8, 82.0, 75.6, 75.1, 67.9, 66.6, 54.2, 51.0, 44.3, 43.7, 31.4. 30.3, 30.1, 29.3, 28.1, 28.0, 26.5, 26.3, 26.1; High Resolution Cl MS *m/z* calcd. for C₂₄H₄₂O₅Cl (MH⁺) 445.2720, found 445.2716.

### EXAMPLE 2: SYNTHESIS OF COMPOUND VI

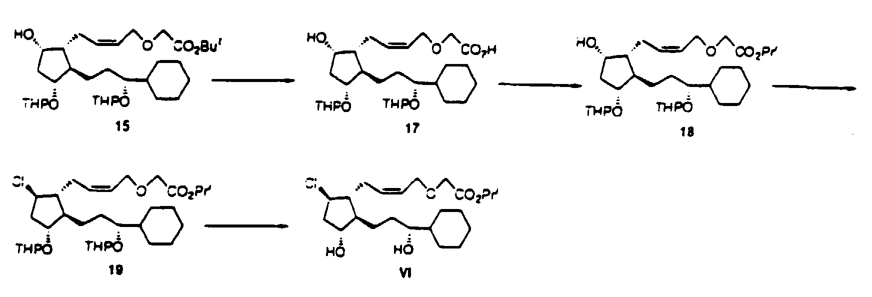

### A: (5Z)-(9S, 11R, 15R)-11,15-Bis-(tetrahydropyran-2-yloxy)-15-cyclohexyl-9-hydroxy-3-oxa-16,17,18,19,20-pentanor-5-prostenoic Acid (17):

Hydroxyester **15** (0.454 g; 0.76 mmol; see Example 1) was dissolved in 10 ml of methanol and 2 ml of water. Lithium hydroxide monohydrate (0.16 g; 500 mol%) was added and the mixture was stirred at room temperature. After 18 h, 20 ml of saturated, aqueous KH₂PO₄ and 20 ml CH₂Cl₂ were added, the layers were separated, and the aqueous phase was washed with additional CH₂Cl₂ (3 X 20 ml). The combined organic layers were dried over Na₂SO₄, filtered, and concentrated. affording 0.47 g of a colorless oil which was used directly in the next reaction.

### B: (5Z)-(9S, 11R, 15R)-11,15-Bis-(tetrahydropyran-2-yloxy-15-cyclohexyl-9-hydroxy-3-oxa-16,17,18,19,20-pentanor-5-prostenoic Acid Isopropyl Ester (18):

Crude acid **17** from above (0.23 g; 0.43 mmol) was dissolved in 10 ml of acetone. DBU (0.25 ml; 400 mol%) and isopropyl iodide (0.21 g; 300 mol%) were added and the mixture was stirred for 12 h at room temperature. After evaporation, the residue was applied to a silica gel column and eluted with hexane/EtOAc, 1/1, to afford 0.157 g (63%) of isopropyl ester **18** as a colorless oil (R_{*f*} =0.49). ¹H NMR (CDCl₃) δ (characteristic peaks only) 5.80-5.52 (m, 2H), 5.15 (sep, 1H, *J*=6.2 Hz), 4.03 (broad s, 2H), 1.27 (d, 6H, *J*=6.2 Hz).

### C: (5Z)-(9R, 11R, 15R)-9-Chloro-15-cyclohexyl-11,15-dihydroxy-3-oxa-l6,17,18,19,20-pentanor-5-prostenoic Acid Isopropyl Ester (VI):

The hydroxyester **18** (0.146 g; 0.25 mmol) was dissolved in 3.0 ml of a stock solution containing 48 ml of CH₃CN, 0.79 ml of pyridine, and 0.97 ml of CCl₄. Triphenvlphosphine (0.10 g; 150 mol%) was added and the resulting mixture was stirred at room temperature for 17 h. The reaction mixture was treated with 10 ml of a 1:1 solution of Et₂O/hexanes and the precipitate was filtered off. The filtrate was concentrated and chromatographed on silica gel (hexane/EtOAc, 4/1), affording 0.108 g of a colorless oil which consisted of a nearly equimolar mixture of desired chlorinated material **19** with its undesired 5,8-diene elimination product

A solution of crude **19** from above in 10 ml of 65% (v/v) aqueous acetic acid was warmed to 65°C for 45 min. The mixture was then cooled to room temperature and concentrated. The resulting residue was then purified by silica gel chromatography (hexane/EtOAc, 2/3), affording 27 mg (25% based on **18**) of pure **VI** (R_{*f*} =0.56) as a colorless oil, and 69 mg of a mixture of **VI** and its 5.8-diene elimination product (R_{*f*} =0.45). ¹H NMR (CDCl₃) δ 5.67 (m, 2H), 5.08 (sep, 1H, *J*=6.1), 4.30-3.95 (m, 6H), 3.40 (m, 1H), 2.35 (m. 2H), 2.30-2.00 (m, 3H), 1.93-1.35 (m, 12H), 1.25 (d, 6H, *J*=6.2 Hz), 1.22-0.90 (m, 6H);¹³C NMR (CDCl₃) δ 170.2, 131.8, 126.7, 75.7, 75.2, 68.8, 67.6, 66.7, 61.2, 54.2, 51.1, 44.4, 43.6, 31.4, 30.2, 30.1, 29.3, 28.0, 26.5, 26.3, 26.1, 21.8; High Resolution CI MS *m/z* calcd. for C₂₃ H₄₀O₅Cl (MH⁺) 431.2564. found 431.2569.

### EXAMPLE 3: SYNTHESIS OF COMPOUND VII

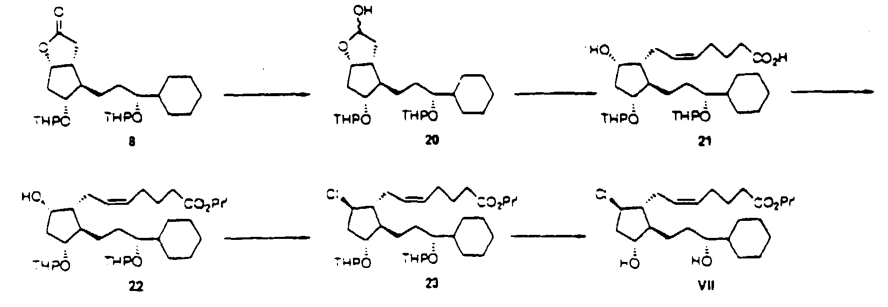

### A: (5Z)-(9S, 11R, 15R)-11,15-Bis-(tetrahydropyran-2-yloxy)-15-cyclohexyl-9-hydroxy-16,17,18,19,20-pentanor-5-prostenoic Acid Isopropyl Ester (22):

To a solution of 5.0 g (11 mmol) of lactone 8 (see Example 1) in 40 ml of THF at -78°C was added dropwise 9.6 ml (14.4 mmol) of a 1.5 *M* solution of diisobutylaluminum hydride in toluene. After 1.5 h, 5 ml of MeOH was added, the mixture was stirred for 10 min at -78°C and then warmed to room temperature. This solution was then added to a mixture of 20 ml of saturated, aqueous NH₄Cl, 35 ml of EtOAc, and 35 ml of saturated, aqueous sodium potassium tartrate. The mixture was stirred for 20 min, lavers were separated, and the aqueous layer was washed with EtOAc (3 X 40 ml). The combined organic layers were dried over MgSO₄, filtered, and evaporated. The resulting residue was purified by silica gel chromatography (EtOAc/hexane, 1/1), affording 4.5 g (90%) of lactol **20** which was used directly in the next reaction.

To a solution of 14.1 g (31.8 mmol) of (1-carboxypent-5-yl)triphenyl-phosphonium bromide in 100 ml of THF at 0°C was added dropwise 59 ml (59 mmol) of a 1 *M* solution of potassium *t*-butoxide in THF. After 20 min, 4.5 g (9.9 mmol) of lactol **20** in 20 ml of THF was added dropwise. The reaction was quenched after 2 h by pouring into 150 ml of a 1/1 (v/v) mixture of EtOAc/ saturated, aqueous NH₄Cl. The layers were separated and the aqueous layer was extracted with EtOAc (3 X 70 ml). The combined organic layers were dried over MgSO_{4,} filtered and evaporated, leaving 7.6 g of crude acid **21** as an oil.

Crude acid **21** (7.6 g) was dissolved in 55 mL of acetone, cooled to 0 °C, and 8.6 g (56 mmol) of DBU was added dropwise. The reaction was warmed to room temperature and 8.5 g (50 mmol) of isopropyl iodide was added dropwise. After stirring for 14 h, the mixture was poured into 100 ml of a 1/1 (v/v) mixture of EtOAc/saturated, aqueous NH₄Cl. The layers were separated and the aqueous phase was extracted with additional EtOAc (2 X 100 ml). The combined organic layers were dried over MgSO₄ filtered, evaporated, and chromatographed on silica gel (EtOAc/hexane, 2/3) affording 1.98 g (35% from lactol **20**) of **22**. ¹H NMR (CDCl₃) δ (characteristic peaks only) 5.58-5.28 (m, 2H), 4.97 (sep, *J*=6.2 Hz, 1H), 1.1 (d, *J*=6.2 Hz, 6 H).

### B: (5Z)-(9R, 11R, 15R)-9-Chloro-15-cyclohexyl-11,15-dihydroxy-16,17,18,19,20-pentanor-5-prostenoic Acid Isopropyl Ester (VII):

Hydroxyester **22** (513.1 mg; 0.8864 mmol) was dissolved in 6.6 ml. of a stock solution containing 48.0 ml of CH₃CN, 0.79 ml of pyridine, and 0.97 ml of CCl₄. Triphenylphosphine (348.8 mg; 150 mol%) was added and the mixture was stirred for 45 h at room temperature. The reaction was then diluted with 7 ml of Et₂O and 14 ml of hexane. After stirring for 10 min. solids were filtered off and the filtrate was evaporated. The resulting solids were triturated three times with 15 mL of hexane/Et₂O (1/2). Combined hexane/Et₂O washes were concentrated down to 0.75 g of a white solid which was then redissolved in hexane/Et₂O and chromatographed on silica gel. Elution with hexane/Et₂O, 5/1, afforded 372.8 mg of semipure **23** which was used directly in the next reaction.

Crude **23** from above was dissolved in 20 ml of 65% (v/v) aqueous HOAc and warmed to room temperature. After 1.5 h, the reaction was concentrated, 15 ml of H₂O was added, and the solution was reconcentrated. Absolute EtOH (15 ml) was added followed, again, by reconcentration. The resulting oil was purified by silica gel chromatography (hexane/EtOAc, 2/1), affording 244.1 mg of a mixture of **VII** contaminated with an approximately equal quantity of the 5,8-diene side product. An 8.2 mg sample was then further purified by reverse phase HPLC, giving 4.4 mg of pure **VII** as a dear, viscous oil. ¹H NMR (CDCl₃) δ 5.47 (m, *J*=8.5 Hz, 2H), 5.01 (sep, *J*=6.3Hz, 1H), 4.10 (dt, *J*=4.0, 6.2 Hz, 1H), 4.04 (q, *J*=7.6 Hz, 1H), 3.37 (m, 1H), 2.35-2.24 (m, 4H), 2.20-2.07 (m, 4 H), 1.82 (br s, 2H), 1.80-1.50 (m, 13H), 1.36-0.96 (m, 12H). ¹³C NMR (CDCl₃) δ 173.2, 131.0, 126.8, 76.2, 76.0, 67.5, 60.8, 54.3, 51.7, 44.5, 43.5, 34.0, 31.7, 30.0, 29.2 (two overlapping resonances), 27.9, 26.6, 26.4, 26.2, 26.1, 24.9, 21.8. High Resolution CI MS *m/z* calcd. for C₂₄H₄₂O₄Cl (MH⁺) 429.2772, found 429.2763.

### EXAMPLE 4: SYNTHESIS OF COMPOUND VIII

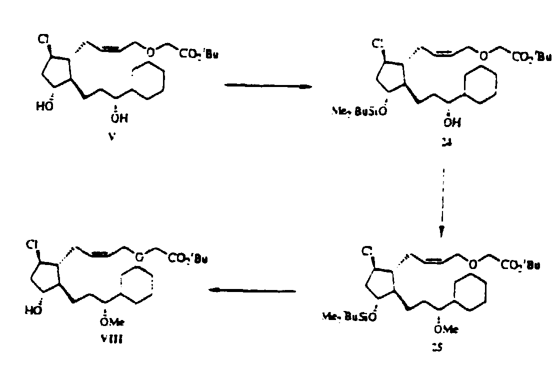

### A: (5Z)-(9R, 11R, 15R)-11-(t-Butyldimethylsiloxy)-9-chloro-15-cyclohexyl-15-hydroxy-3-oxa-16,17,18,19,20-pentanor-5-prostenoic acid t-butyl ester (24):

To a mixture of 127 mg (0.285 mmol) of Compound **V** (Example 1), 49 mg (0.72 mmol) of imidazole, 10 mg (0.082 mmol) of 4-(dimethylamino)pyridine (DMAP), and 5 ml of CH₂Cl₂ was added 90 mg (0.59 mmol) of *tert*butyldimethylsilyl chloride. After stirring overnight, 10 ml of saturated NH₄Cl was added, the mixture was extracted with CH₂Cl₂ (3 X 10 ml), the combined organic layers were dried over MgSO₄, filtered and concentrated, and the residue was chromatographed on silica gel (20% ethyl acetate in hexane) to afford 87 mg (55%) of **24**.

### B: (5Z)-(9R, 11R, 15R)-11-(t-Butyldimethylsiloxy)-9-chloro-15-cyclohexy-15-methoxy-3-oxa-16,17,18,19,20-pentanor-5-prostenoic acid t-butyl ester (25):

A mixture of 80 mg (0.14 mmol) of **24**, 100 mg (0.52 mmol) of 2,6-di-*t*-butylpyridine, 80 mg (0.51 mmol) of methyl trifluoromethanesulfonate, and 2 ml of CH₂Cl₂ was refluxed overnight. The reaction was cooled to room temperature, poured into 10 mL of saturated NaHCO₃, extracted with CH₂Cl₂ (3 X 10 ml), the combined organic layers were dried over MgSO₄, filtered, and concentrated, and the residue was chromatographed on silica gel (10% ethyl acetate in hexane) to afford 35 mg (44%) of **25**.

### C: (5Z)-(9R, 11R, 15R)-9-Chloro-15-cyclohexyl-11-hydroxy-15-methoxy-3-oxa-16,17,18,19,20-pentanor-5-prostenoic acid t-butyl ester (VIII):

To a mixture of 32 mg (0.056 mmol) of **25** and 1.5 ml of THF was added 0.12 ml (0.12 mmol) of 1 *M* tetrabutylammonium fluoride (TBAF) in THF. After 30 min, 4 ml of saturated NH₄Cl was added, the mixture was extracted with ethyl acetate (3 X 5 ml), the combined organic layers were dried over MgSO₄, filtered, and concentrated, and the residue was chromatographed on silica gel (40% ethyl acetate in hexane) to afford 24 mg (93%) of **VIII**. ¹³C NMR (CDCl₃) δ 169.77 (C), 130.90 (CH), 127.43 (CH), 85.89 (CH), 81.69 (C), 76.05 (CH), 67.83 (CH₂), 66.56 (CH₂), 61.00 (CH), 57.83 (CH₃), 54.06 (CH), 51.92 (CH), 44.45 (CH₂), 40.65 (CH), 29.92 (CH₂), 29.83 (CH₂), 29.02 (CH₂), 28.42 (CH₃), 28.10 (CH₂), 26.64 (CH₂), 26.37 (CH₂). CI MS, *m/z* calcd. for C₂₅H₄₄O₅Cl (MH⁺) 459.2877, found 459.2877.

### EXAMPLE 5: SYNTHESIS OF COMPOUND IX

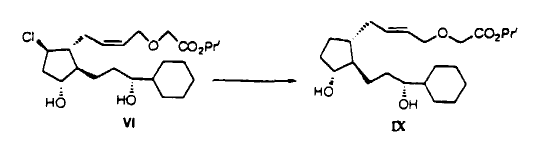

### A: (5E)-(11R, l5R)-15-Cyclohexyl-11.15-dihydroxy-3-oxa-16,17,18,19,20-pentanor-5-prostenoic acid isopropyl ester (IX):

To a solution of the ester **VI** (390 mg, 0.90 mmol) (see example 2) and AIBN (10 mg) in anhydrous toluene (9.0 ml) was added *n*-Bu₃SnH (0.47 ml, 1.80 mmol). The resulting mixture was heated at reflux for 4 h. The solvent was evaporated and the residue was applied to a silica gel column for purification. The product **IX** (R_{*f*}=0.4, 60% EtOAc/hexane) was isolated as a colorless oil (340 mg isolated, 95% yield). ¹H NMR (CDCl₃) δ 5.60 (m, 2H), 5.05 (septet, *J* = 6.5 Hz, 1H), 3.98 (m, 4H), 3.85 (m, 1H), 3.30 (m, 1H), 2.22 (m, 1H), 2.00 (m, 1H), 1.80 - 0.85 (broad m, 23H), 1.21 (d, *J* = 6.4 Hz, 6H); ¹³C NMR (CDCl₃) δ 170.11, 134.59, 126.54, 79.15, 76.35, 72.07, 68.50, 67.18, 53.57, 44.21, 43.62, 38.21, 34.19, 32.05, 29.47, 29.32, 28.94, 27.98, 26.56, 26.36, 26.21, 21.84.

### EXAMPLE 6: SYNTHESIS OF COMPOUND X

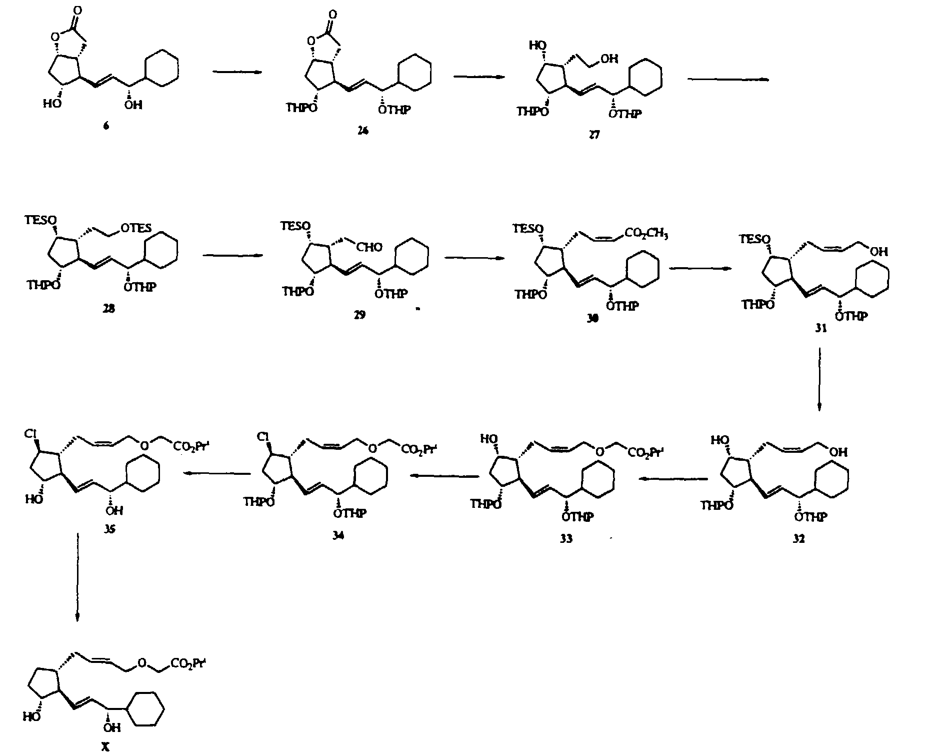

### A: (3aR, 4R, 5R, 6aS)-4-[(E)-(3S)-3-Cyclohexyl-3-(tetrahydropyran-2-yloxy)propenyl]-hexahydro-5-(tetrahydropyran-2-yloxy)-2H-cyclopenta[b]furan-2-one (26):

To 15.7 g (55.9 mmol) of diol 6 (Example 1) in 120 ml of CH₂Cl₂ at 0°C was added 12.0 g (142 mmol) of 3,4-dihydro-2H-pyran (DHP) and 520 mg (2.7 mmol) of ρ-toluenesulfonic acid monohydrate (pTSA). After 1 h at 0°C, 100 ml of saturated NaHCO₃ was added, the mixture was extracted with CH₂Cl₂ (3 X 100 ml), the combined organic layers were dried over MgSO₄, filtered and concentrated and the residue was chromatographed on silica gel (40% ethyl acetate in hexane) to afford 13.3 g (53%) of bis-THP ether **26**.

### B: (13E)-(9S,11R,15S)-11,15-Bis(tetrahydropyran-2-yloxy)-15-cyclohexyl-2,3,4,5,6,16,17,18,19,20-decanor-9-hydroxy-13-prostenol (27):

To a solution of 15.0 g (33.4 mmol) of **26** in 150 ml of THF at 0°C was added 53 ml (80 mmol) of 1.5 *M* solution of diisobutylaluminum hydride (DIBAL-H) in toluene. After 3 h the reaction was poured into 300 ml of 1:1 ethyl acetate:saturated sodium potassium tartrate and stirred for 1 h. The layers were separated, the aqueous layer was extracted with ethyl acetate (3 X 100 ml), the combined organic layers were dried over MgSO₄, filtered and concentrated to afford 14.89 g (98%) of crude diol **27**.

### C: (13E)-(9S,11R,15S)-11,15-Bis(tetrahydropyran-2-yloxy)-15-cyclohexyl-2,3,4,5,6,16,17,18,19,20-decanor-9-(triethylsiloxy)-13-prostenol triethylsilyl ether (28):

To a mixture of 14.8 g (32.7 mmol) of **27**, 5.94 g (87.4 mmol) of imidazole, 0.44 g (3.6 mmol) of DMAP, and 150 ml of CH₂Cl₂ was added 11.5 g (76.3 mmol) of triethylsilyl chloride. After 5 h, 150 m 1 of saturated NH₄Cl was added, the layers were separated, and the aqueous layer was extracted with CH₂Cl₂ (2 X 100 ml). The combined organic layers were dried over MgSO₄, filtered, and concentrated, and the residue was chromatographed on silica gel (10% ethyl acetate in hexane) to afford 21.9 g (100%) of bis-silyl ether **28**.

### D: (13E)-(9S,11R,15S)-11,15-Bis(tetrahydropyran-2-yloxy)-15-cyclohexyl-2,3,4,5,6,16,17,18,19,20-decanor-9-(triethylsiloxyl-13-prostenal (29):

To 12.5 g (98.6 mmol) of oxalyl chloride in 150 mL of CH₂Cl₂ at -78°C was added dropwise a solution of DMSO (13.0 g, 166 mmol) in 15 ml of CH₂Cl₂. After 30 min, a solution of **28** (22.4 g, 32.9 mmol) in 60 ml of CH₂Cl₂. After 5 h, 36 g (360 mmol) of NEt₃ was added, and the reaction was stirred for 30 min at -78°C and then at room temperature for 30 min. The mixture was poured into 200 mL of saturated NH₄Cl and extracted with CH₂Cl₂ (3 X 150 ml), the combined organic layers were dried over MgSO₄, filtered, and concentrated, and the residue was chromatographed on silica gel (20% ethyl acetate in hexane) to afford 18.3 g (99%) of aldehyde **29**.

### E: (5Z, 13E)-(9S,11R, 15S)-11,15-Bis(tetrahydropyran-2-yloxy)-15-cyclohexyl)-2,3,4,16,17,18,19,20-octanor-9-(triethylsiloxy-5,13-prostadienoic acid methyl ester (30):

To a mixture of 16.5 g (51.9 mmol) of bis(2,2,2-trifluoroethyl) (methoxycarbonylmethyl)phosphonate, 28.2 g (107 mmol) of 18-crown-6, and 200 mL of THF at -78°C was added dropwise 85 ml (42.5 mmol) of 0.5 *M* KHMDS in toluene. After 30 min, a solution of 18.3 g (32.4 mmol) of **29** in 50 ml of THF was added dropwise and the reaction stirred for 2 h. The mixture was poured into 150 mL of saturated NH₄Cl, extracted with ethyl acetate (3 X 100 ml), the combined organic layers were dried over MgSO₄, filtered and concentrated, and the residue was chromatographed on silica gel (15% ethyl acetate in hexane) to afford 9.78 g (49%) of crotonate **30**, as well as 3.58 g (18%) of a mixture of olefin *cis:trans* isomers.

### F: (5Z, 13E)-(9S,11R, 15S)-11,15-Bis(tetrahydropyran-2-yloxy)-15-cyclohexyl-2,3,4,16,17,18,19,20-octanor-9-(triethylsiloxy)-5,13-prostadienol (31):

To a solution of 9.11 g (14.7 mmol) of **30** in 40 mL of THF at 0°C was added dropwise 24 ml (36 mmol) of a 1.5 *M* solution of DIBAL-H in toluene. After 1 h, the reaction was added to 100 ml of saturated NH₄Cl, extracted with ethyl acetate (3 X 75 ml), the combined organic layers were dried over MgSO₄, filtered, and concentrated to afford 8.7 g (100%) of crude allyl alcohol **31**.

### G: (5Z, 13E)-(9S,11R, 15S)-11,15-Bis(tetrahydropyran-2-yloxy)-15-cyclohexyl-9-hydroxy-2,3,4,16,17,18,19,20-octanor-5,13-prostadienol (32):

To a solution of 8.7 g (14.7 mmol) of **31** in 60 ml of THF at 0°C was added 20 mL (20 mmol) of a 1 *M* solution of TBAF in THF. After lh, 75 ml of saturated NH₄Cl was added, the mixture was extracted with ethyl acetate (3 X 75 ml), the combined organic layers were dried over MgSO₄, filtered, and concentrated, and the residue was chromatographed on silica gel (3/2 ethyl acetate/hexane) to afford 3.79 g (54%) of diol **32**.

### H: (5Z, 13E)-(9S,11R, 15S)-11,15-Bis(tetrahydropyran-2-yloxy)-15-cyclohexyl-9-hydroxy-3-oxa-16,17,18,19,20-pentanor-5,13-prostadienoic acid isopropyl ester (33):

A mixture of **32** (3.76 g, 7.7 mmol), NaOH (6.1 g, 150 mmol), water (30 ml), toluene (30 ml, tetrabutylammonium hydrogen sulfate (1.05 g, 3.1 mmol), and isopropyl bromoacetate (3.63 g, 20.1 mmol) at room temperature was vigorously stirred for 30 min. The layers were separated, the aqueous layer was extracted with ethyl acetate (2 X 30 m l), the combined organic layers were dried over MgSO₄, filtered and concentrated, and the residue was chromatographed on silica gel (40% ethyl acetate in hexane) to afford ester **33** (3.06 g, 69%).

### I: (5Z, 13E)-(9R,11R, 15S)-11,15-Bis(tetrahydropyran-2-yloxy)-9-chloro-15-cyclohexyl-3-oxa-16,17,18,19,20-pentanor-5,13-prostadienoic acid isopropyl ester (34):

To a mixture of **33** (3.0 g, 5.2 mmol) and 30 ml of pyridine at 0°C was added methanesulfonyl chloride (1.48 g, 12.9 mmol). The reaction was brought to room temperature after 15 min, stirred for an additional 1.5 h, and poured into a suspension of Bu₄NCl (14.5 g, 52.2 mmol) in 45 ml of toluene. The mixture was stirred at room temperature overnight, at 55-60°C for 2 h, poured into 100 ml of saturated NH₄Cl, extracted with ethyl acetate (3 X 75 ml), the combined organic layers were dried over MgSO₄, filtered, and concentrated, and the residue was chromatographed on silica gel (30% ethyl acetate in hexane) to afford 3.0 g of an oil which consisted of a mixture of chlorinated compound **34** and the 8,9-olefin elimination by-product. This mixture was used in the next step without further purification.

### J: (5Z,13E)-(9R,11R, 15S)-9-Chloro-15-cyclohexyl-11,15-dihydroxy-3-oxa-16,17,18,19,20-pentanor-5,13-prostadienoic acid isopropyl ester (35):

The sample from above (3.0 g) was dissolved in 40 ml of isopropyl alcohol and 5 ml of water was added, followed by 2.3 ml of 12 *M* HCI. After 3 h, saturated NaHCO₃ was added (50 ml), the mixture was extracted with ethyl acetate (3 X 50 ml), the combined organic layers were dried over MgSO₄, filtered, and concentrated, and the residue was purified by radial chromatography (20/1 toluene/isopropanol) to afford 324 mg of pure chloride **35** (32%), as well as 278 mg (28%, calculated as the chloride) of a mixture of **35** with the 8,9-olefin. ¹³C NMR (CDCl₃) δ 170.04 (C), 134.51 (CH), 132.64 (CH), 130.43 (CH), 127.58 (CH), 77.34 (CH), 75.40 (CH), 68.60 (CH), 67.60 (CH₂), 66.64 (CH₂), 59.64 (CH), 55.95 (CH), 53.36 (CH), 43.61 (CH), 43.57 (CH₂), 28.83 (CH₂), 28.70 (CH₂), 26.49 (CH₂), 26.06 (CH₂), 25.99 (CH₂), 21.82 (CH₃). CI MS, *m/z* calcd. for C₂₃H₃₈O₅Cl (MH⁺) 429.2408, found 429.2408.

### K: (5E, 13E)-(11R, 15S)-15-Cyclohexyl-11,15-dihydroxy-3-oxa-16,17,18,19,20-pentanor-5,13-prostadienoic acid isopropyl ester (X):

A mixture of **35** (60 mg, 0.14 mmol), benzene (3 ml), AIBN (10 mg), and Bu₃SnH (90 mg, 0.31 mmol) was deoxygenated by purging with N₂ for 15 min, and heated at reflux for 1 h. The reaction was concentrated and chromatographed on silica gel (3:2 ethyl acetate:hexane) to afford 43 mg (78%) of dechlorinated product **X** ¹³C NMR (CDCl₃) δ 169.98 (C), 134.27 (CH), 134.22 (CH), 133.94 (CH), 126.62 (CH), 77.76 (CH), 77.72 (CH), 71.92 (CH₂), 68.41 (CH), 67.06 (CH₂), 58.01 (CH), 43.33 (CH), 42.18 (CH), 36.99 (CH₂), 32.02 (CH₂), 28.94 (CH₂), 28.72 (CH₂), 27.38 (CH₂), 26.48 (CH₂), 26.03 (CH₂), 25.96 (CH₂), 21.74 (CH₃). CI MS, *m/z* calcd. for C₂₃H₃₉O₅ (MH⁺) 395.2798, found 395.2798.

### EXAMPLE 7: SYNTHESIS OF COMPOUND XI

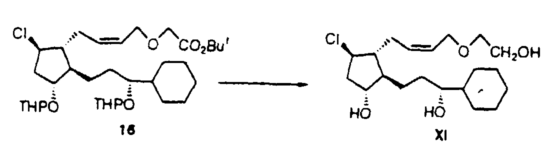

### (5Z)-(9R,11R, 15R)-9-Chloro-15-cyclohexyl-11,15-dilhydroxy-3-oxa-16,17,18,19,20-pentanor-5-prostenol (XI):

A solution of the ester **16** (150 mg, 0.24 mmol; Example 1) in THF (5.0 ml) was cooled to 0°C and to it DIBAL-H (1.5 *M* in toluene, 0.5 ml, 0.72 mmol) was added and the resulting mixture was stirred at 0°C for 2.5 h. The reaction was carefully quenched with a saturated solution of potassium sodium tartrate (10 ml) and the biphasic mixture was stirred at ambient temperature for lh. The organic layer was then separated and the aqueous layer was extracted with EtOAc (5 X 10 ml), The combined organic layers were dried (Na₂SO₄), filtered, and concentrated to afford a pale yellow liquid which was purified by chromatography on silica gel. The intermediate alcohol (R_{*f*}=0.15, 30% EtOAc/hexanes) was isolated (114 mg, 87% yield) as a colorless oil and was used in the subsequent reaction.

The above alcohol (54 mg, 0.09 mmol) was mixed with 65% acetic acid/water (15 ml) and then heated at 70°C for 1 h. The solvent was evaporated and the crude was applied to a column of silica gel for purification. The triol **XI** (R_{*f*}=0.15 EtOAc) was isolated as a colorless oil (33 mg, 88% yield). ¹H NMR (CDCl₃) δ 5.68 (m, 2H), 4.08 (m, 4H), 3.74 (m, 2H), 3.57 (m, 2H), 3.42 (m, 1H), 2.37 (m, 2H), 2.35-1.90 (m, 4H), 1.85-0.90 (broad m, 18H); ¹³C NMR (CDCl₃) δ 131.06, 127.52, 75.80, 75.54, 71.94, 66.38, 61.82, 60.82, 54.05, 50.87, 44.69, 43.60, 31.29, 29.71, 29.46, 29.22, 28.08, 26.48, 26.28, 26.14; CI MS calcd. for C₂₀H₃₆O₄Cl (MH⁺) 375.2302, found 375.2299.

### EXAMPLE 8: SYNTHESIS OF COMPOUND XII

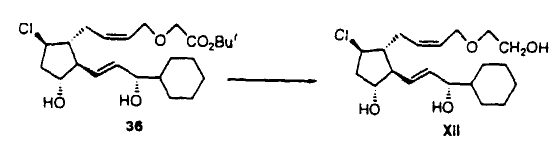

### (5Z,13E)-(9R,11R, 15S)-9-Chloro-15-cyclohexyl-11,15-dihydroxy-3-oxa-16,17,18,19,20-pentanor-5,13-prostadienol (XII):

To a solution of ester **36** (41 mg, 0.093 mmol) (German patent DE 3724189) in 5 ml of THF at 0°C was added a 1.5 *M* solution of DIBAL-H (0.7 ml 1.05 mmol). After warming to room temperature and stirring for 1.5 h, 15 ml of saturated NH₄Cl was added, the mixture was extracted with ethyl acetate (3 X 15 ml), the combined organic layers were dried over MgSO₄, filtered, and concentrated, and the residue was chromatographed on silica gel (ethyl acetate) to afford triol **XII** (21 mg, 68%). ¹³C NMR (CDCl₃) δ 134.59 (CH), 133.00 (CH), 129.61 (CH), 128.21 (CH), 77.56 (CH), 75.16 (CH), 71.54 (CH₂), 67.93 (CH₂), 66.46 (CH₂), 61.76 (CH₂), 59.49 (CH), 55.85 (CH), 53.23 (CH), 43.43 (CH), 28.81 (CH₂), 28.56 (CH₂), 26.46 (CH₂), 26.02 (CH₂), 26.94 (CH₂), 25.57 (CH₂). CI MS, *m/z* calcd. for C₂₀H₃₄O₄Cl (MH⁺) 373.2146, found 373.2101.

### EXAMPLE 9: SYNTHESIS OF COMPOUND XIII

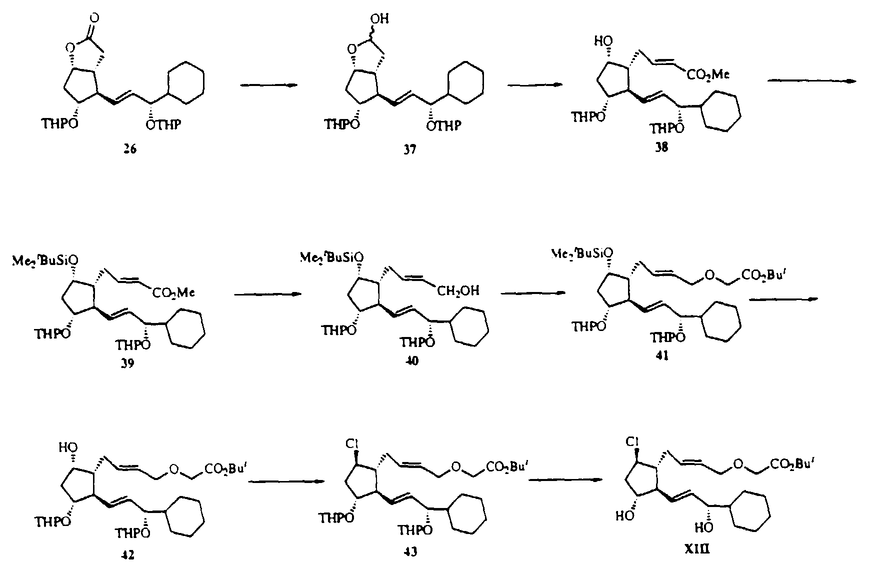

### A: (3aR, 4R, 5R, 6aS)-4-[(E)-(3S)-3-Cyclohexyl-3-(tetrahydropyran-2-yloxylpropenyl]-hexahydro-5-(tetrahydropyran-2-yloxy)-2H-cyclopenta[b]furan-2-ol (37):

To a solution of lactone **26** (5.7 g, 12.7 mmol; Example 6) in 40 ml of THF at -78°C was added dropwise a 1.5 *M* solution of DIBAL-H in toluene (11.5 ml, 17.2 mmol). After 2 h, the reaction was poured into 70 ml of a saturated solution of sodium potassium tartrate and was stirred for 30 min. The mixture was extracted with ethyl acetate (3 X 50 ml), the combined organic layers were dried over MgSO₄, filtered and concentrated, and the residue was chromatographed on silica gel (1/1 hexane/ethyl acetate) to afford 4.7 g (82%) of lactol **37**.

### B: (5E, 13E)-(9S, 11R, 15S)-11,15-Bis(tetrahydropyran-2-yloxy)-15-cyclohexyl-9-hydroxy-2,3,4,16,17,18,19,20-octanor-5,13-prostadienoic acid methyl ester (38):

A mixture of **37** (5.1 g, 11.3 mmol), Ph₃PCH=CO₂Me (6.6 g, 19.7 mmol), CH₂Cl₂ (50 m l), and acetic acid (8 drops) was stirred overnight at room temperature. The mixture was concentrated and chromatographed on silica gel (1/1 hexane/ethyl acetate) to afford 5.7 g (99%) of *trans*-crotonate **38**.

### C: (5E, 13E)-(9S, 11R, 15S)-11,15-Bis(tetrahydropyran-2-yloxy)-9-(t-butyldimethylsiloxy)-15-cyclohexyl-2,3,4,16,17,18,19,20-octanor-5,13-prostadienoic acid methyl ester (39):

A mixture of **38** (5.7 g, 11.6 mmol), CH₂Cl₂ (150 ml), imidazole (1.46 g, 21.5 mmol), DMAP (500 mg, 4.1 mmol), and *t*-butyldimethylsilyl chloride (2.54 g, 16.9 mmol) was stirred for 1 h, 50 ml of saturated NH₄Cl was added, the layers were separated, the aqueous layer was extracted with CH₂Cl₂ (2 X 50 m l), the combined organic layers were dried over MgSO₄, filtered and concentrated, and the residue was chromatographed on silica gel (20% ethyl acetate in hexane) to afford **39** (6.05 g, 84%).

### D: (5E, 13E)-(9S, 11R, 15S)-11,15-Bis(tetrahydropyran-2-yloxy)-9-(t-butyldimethylsiloxyl-15-cyclohexyl-2,3,4,16,17,18,19,20-octanor-5,13-prostadienol (40):

To a solution of **39** (6.0 g, 9.8 mmol) in THF (50 ml) at 0°C was added dropwise a 1.5 *M* solution of DIBAL-H in toluene (16 mL, 24 mmol). The reaction was brought to room temperature and was stirred for 2 h, 75 ml of a saturated solution of sodium potassium tartrate was added, and the mixture was stirred for 35 min. The layers were separated, the aqueous layer was extracted with ethyl acetate (2 X 50 ml), the combined organic layers were dried over MgSO₄, filtered, and concentrated, and the residue was chromatographed on silica gel (25% ethyl acetate in hexane) to afford **40** (4.28 g, 74%).

### E: (5E, 13E)-(9S,11R, 15S)-11,15-Bis(tetrahydropyran-2-yloxy)-9-(t-butyldimethylsiloxy)-l5-cyclohexyl-3-oxa-16,17,18,19,20-pentanor-5,13-prostadienoic acid t-butyl ester (41):

A mixture of **40** (2.4 g, 4.1 mmol), water (25 ml), toluene (30 ml), NaOH (3.8 g, 95 mmol), Bu₄NHSO₄ (300 mg, 0.88 mmol), and *t*-butyl bromoacetate (5.0 g, 25.6 mmol) was stirred vigorously overnight. The layers were separated, the aqueous layer was extracted with ethyl acetate (2 X 50 ml), the combined organic layers were dried over MgSO₄, filtered and concentrated, and the residue was chromatographed on silica gel (20% ethyl acetate in hexane) to afford **41** (1.48 g, 48%).

### F: (5E, 13E)-(9S, 11R, 15S)-11,15-Bis(tetrahydropyran-2-yloxy)-15-cyclohexyl-9-hydroxy-3-oxa-16,17,18,19,20-pentanor-5,13-prostadienoic acid t-butyl ester (42): A

mixture of **41** (1.4 g, 2.0 mmol), THF (20 ml), and a 1 *M* solution of TBAF in THF (6 mL, 6 mmol) was stirred for 2 h, saturated NH₄Cl was added (30 ml), the layers were separated, the aqueous layer was extracted with ethyl acetate (2 X 40 ml), the combined organic layers were dried over MgSO₄, filtered, and concentrated, and the residue was chromatographed on silica gel (1/1 hexane/ethyl acetate) to afford **42** (0.45 g, 38%).

### G: (5E, 13E)-(9R, 11R, 15S)-11,15-Bis(tetrahydropyran-2-yloxy)-9-chloro-15-cyclohexyl-3-oxa-16,17,18,19,20-pentanor-5,13-prostadienoic acid t-butyl ester (43):

A mixture of **42** (430 mg, 0.72 mmol), PPh₃ (350 mg, 1.34 mmol), CH₃CN (6 ml), pyridine (112 mg, 1.42 mmol), and CCl₄ (240 mg, 1.55 mmol) was stirred overnight. The reaction was concentrated and the residue was chromatographed on silica gel (20% ethyl acetate in hexane) to afford **43** as a mixture with the corresponding 8,9-olefin (362 mg, 82% calculated as the chloride). This mixture was separated in the next step.

### H: (5E, 13E)-(9R, 11R, 15S)-9-Chloro-15-cyclohexyl-11,15-dihydroxy-3-oxa-16,17,18,19,20-pentanor-5,13-prostadienoic acid t-butyl ester (XIII):

A mixture of **43** (310 mg, 0.51 mmol calculated as the chloride), THF (5 ml), water (1 ml), and acetic acid (9 ml) was heated at 65°C for 1 h. The reaction was concentrated and the residue was chromatographed on silica gel (4/1 ethyl acetate/hexane) to afford **XIII** as a mixture with the corresponding 8,9-olefin (188 mg, 83% calculated as the chloride). The mixture was separated by reverse-phase HPLC to afford pure **XIII** (58 mg, 26% from alcohol **46**). ¹³C NMR (CDCl₃) δ 169.67 (C), 134.67 (CH), 133.09 (CH), 131.18 (CH), 128.56 (CH), 81.62 (C), 77.31 (CH), 75.04 (CH), 71.63 (CH₂), 67.59 (CH₂), 59.38 (CH), 56.34 (CH), 53.08 (CH), 43.32 (CH), 43.38 (CH₂), 33.88 (CH₂), 28.87 (CH₂), 28.77 (CH₂), 28.10 (CH₃), 26.48 (CH₂), 26.05 (CH₂), 25.97 (CH₂). CI MS *m/z* calcd. for C₂₄H₄₀O₅Cl 445.2535, found 445.2574.

### EXAMPLE 10: SYNTHESIS OF COMPOUND VIV

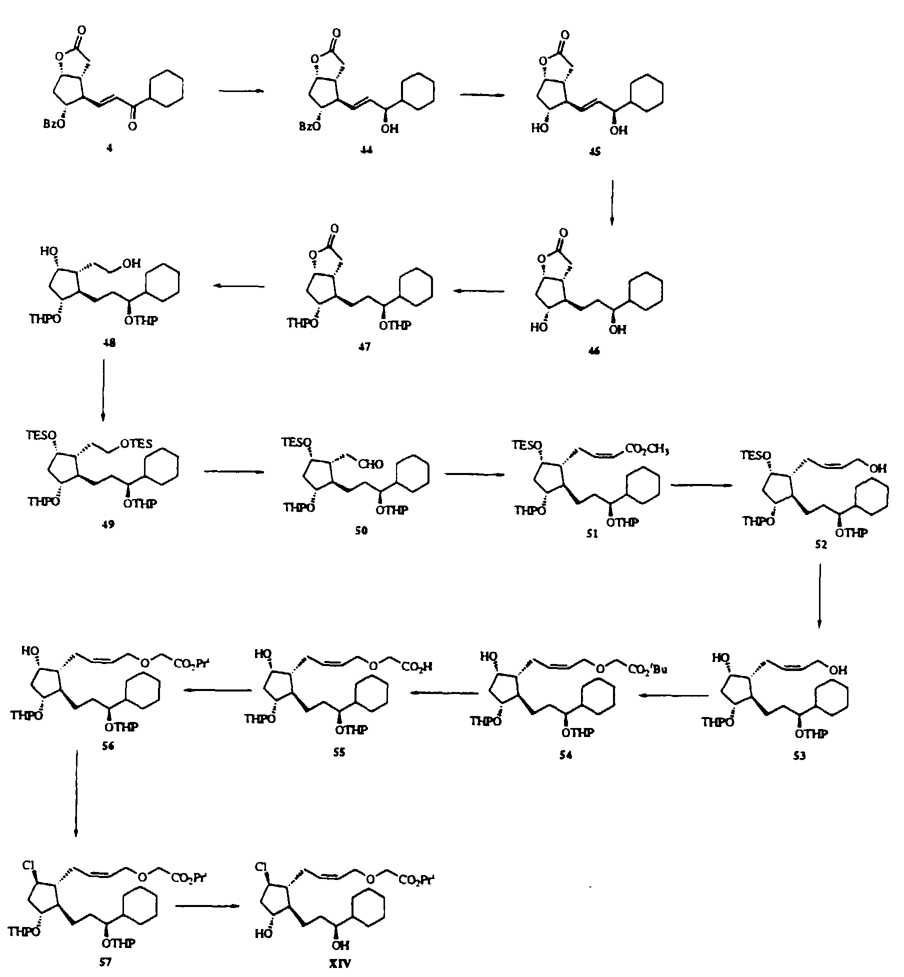

### A: 3aR, 4R, 5R, 6aS)-5-(Benzoyloxy)-4-[(E)-(3R)-3-cyclohexyl-3-hydroxy-1-propenyl]-hexahydro-2H-cyclopenta[b]furan-2-one (44):

To a solution of enone **4** (150 g, 392 mmol; Example 1), cerium trichloride heptahydrate (152 g, 408 mmol), methanol (500 ml), and CH₂Cl₂ (1.5 L) at 0°C was added NaBH₄ (14.4 g) in 0.2 g portions over 1 h. After stirring for 2 h, the reaction was poured into 1 *M* HCl (500 ml), the layers were separated, the aqueous layer was extracted with CH₂Cl₂ (2 X 300 ml), and the combined organic layers were dried over MgSO₄, filtered, and concentrated to provide 150.4 g (100%) of a 1:1 mixture of the two diastereomeric allylic alcohols. Separation of 200 g of the mixture (from several combined reactions) by HPLC (40% ethyl acetate in hexane) afforded 23.7 g (12%) of alcohol **44**.

### B: (3aR, 4R, 5R, 6aS)-4-[(E)-(3R)-3-Cyclohexyl-3-hydroxy-1-propenyl]-hexahydro-5-hydroxy-2H-cyclopental[b]furan-2-one (45):

To a solution of **44** (7.35 g, 19.1 mmol) in methanol (100 m 1) was added K₂CO₃ (2.64 g, 19.1 mmol). After 2 h, 200 ml of 2 *M* HCl was added, the mixture was extracted with ethyl acetate (3 X 100 ml), and the combined organic layers were dried over MgSO₄, filtered, and concentrated to afford crude diol **45** (5.53 g), which was used in the next step without purification.

### C: (3aR, 4R, 5R, 6aS)-4-[(3S)-3-Cyclohexyl-3-hydroxypropyl]-hexahydro-5-hydroxy-2H-cyclopenta[b]furan-2-one (46):

A mixture of **45** (5.3 g, 18.9 mmol), 10% w/w Pd/C (320 mg), ethyl acetate (30 ml), and CH₂Cl₂ (10 ml) was hydrogenated in a Parr hydrogenation apparatus for 3 h at 45 psi. The mixture was filtered through Celite and concentrated, and the residue was chromatographed on silica gel (ethyl acetate) to afford **46** (4.3 g, 80%). ¹³C NMR (CDCl₃) δ 177.64 (C), 83.87 (CH), 77.21 (CH), 76.02 (CH), 53.45 (CH), 43.84 (CH), 42.96 (CH), 40.46 (CH₂), 35.88 (CH₂), 31.37 (CH₂), 29.23 (CH₂), 29.10 (CH₂), 27.85 (CH₂), 26.41 (CH₂), 26.22 (CH₂), 26.08 (CH₂).

### D: (3aR, 4R, 5R, 6aS)-4-[(3S)-3-Cyclohexyl-3-(tetrahydropyran-2-yloxy)propyl]-hexahydro-5-(tetrahydropyran-2-yloxy)-2H-cyclopenta[b]furan-2-one (47):

To a mixture of **46** (3.7 g, 12.9 mmol), DHP (2.9 g, 35 mmol), and CH₂Cl₂ (80 ml) at 0°C was added pTSA (300 mg, 1.58 mmol). After 30 min 50 ml of saturated NaHCO₃ was added, the layers were separated, the aqueous layer was extracted with CH₂CL₂ (2 X 50 ml), the combined organic layers were dried over MgSO₄, filtered, and concentrated, and the residue was chromatographed on silica gel (1/1 hexane/ethyl acetate) to afford bis-THP ether **47** (4.89 g, 89%).

### E: (9S, 11R, 15S)-11,15-Bis(tetrahydropyran-2-yloxy)-15-cyclohexyl-2,3,4,5,6,16,17,18,19,20-decanor-9-hydroxyprostanol (48):

To a suspension of lithium aluminum hydride (920 mg, 24.2 mmol) in 50 ml of THF at 0°C was added dropwise a solution of lactone **47** (10.87 g, 24.1 mmol) in THF (100 ml). After stirring for 1 h, ethyl acetate was cautiously added dropwise (50 ml), followed by saturated NH₄Cl (100 ml). The mixture was extracted with ethyl acetate (3 X 100 ml), the combined organic layers were dried over MgSO₄, filtered, and concentrated to provide crude diol **48** (9.73 g, 89%), which was used in the next step without purification.

### F: (9S, 11R, 15S)-11,15-Bis(tetrahydropyran-2-yloxy)-15-cyclohexyl-2,3,4,5,6,16,17,18,19,20-decanor-9-(triethylsiloxy)prostanol triethylsilyl ether (49):

A mixture of **48** (9.7 g, 21.3 mmol), triethylsilyl chloride (7.36 g, 48.9 mmol), DMAP (289 mg, 2.36 mmol), NEt₃ (5.3 g, 52 mmol), and CH₂Cl₂ (100 ml) was stirred overnight at room temperature. The mixture was poured into 100 ml of saturated NH₄Cl, the layers were separated, the aqueous layer was extracted with CH₂Cl₂ (50 m l), the combined organic layers were dried over MgSO₄, filtered, and concentrated, and the residue was chromatographed on silica gel (15% ethyl acetate in hexane) to afford **49** (11.75 g, 81%).

### G: (9S, 11R,15S)-11,15-Bis(tetrahydropyran-2-yloxy)-15-cyclohexyl-2,3,4,5,6,16,17,18,19,20-decanor-9-(triethylsiloxy)prostanal (50):

To a solution of oxalyl chloride (6.5 g, 51 mmol) in 30 ml of CH₂Cl₂ at -78°C was added dropwise a solution of DMSO (6.7 g, 86 mmol) in 10 ml of CH₂Cl₂. After 30 min, a solution of **49** (11.75 g, 17.2 mmol) in 90 ml of CH₂Cl₂ was added dropwise. After 6 h, NEt₃ (18.9 g, 187 mmol) was added and the reaction was warmed to room temperature. The mixture was added to 150 ml of saturated NH₄Cl, the layers were separated, the aqueous layer was extracted with CH₂Cl₂ (2 X 50 ml), the combined organic layers were dried over MgSO₄, filtered, and concentrated, and the residue was chromatographed on silica gel (10% ethyl acetate in hexane) to afford aldehyde **50** (8.75 g, 90%).

### H: (5Z)-(9S,11R, 15S)-11,15-Bis(tetrahydropyran-2-yloxy)-15-cyclohexyl-2,3,4,16,17,18,19,20-octanor-9-(triethylsiloxy)-5-prostenoic acid methyl ester (51):

To a mixture of 18-crown-6 (13.1 g, 49.6 mmol), bis(2,2,2-trifluoroethyl) (methoxycarbonylmethyl)phosphonate (7.8 g, 24.6 mmol), and 75 ml of THF at -78°C was added dropwise a 0.5 *M* solution of KHMDS in toluene (42 ml, 21.5 mmol). After 30 min, a solution of 50 (8.75 g, 15.4 mmol) in THF (75 ml) was added dropwise. After 2.5 h methanol was added (10 ml), the reaction was warmed to room temperature and added to saturated NH₄Cl (100 ml), the layers were separated, the aqueous layer was extracted with ethyl acetate (2 X 50 ml), the combined organic layers were dried over MgSO₄, filtered, and concentrated, and the residue was chromatographed on silica gel (15% ethyl acetate in hexane) to afford *cis*-crotonate **51** (4.05 g, 44%), as well as a mixture of *cis:trans* olefin isomers (2.3 g, 25%).

### I: (5Z)-(9S,11R, 15S)-11,15-Bis(tetrahydropyran-2-yloxy)-15-cyclohexyl-2,3,4,16,17,18,19,20-octanor-9-(triethylsiloxy)-5-prostenol (52):

To a solution of **51** (4.0 g, 6.4 mmol) in 25 ml of THF at 0°C was added dropwise a 1.5 *M* solution of DIBAL-H in toluene (10 ml, 15 mmol). The reaction was brought to room temperature and stirred overnight. The mixture was added to 75 m l of saturated sodium potassium tartrate and stirred for 30 min, the layers were separated, the aqueous layer was extracted with ethyl acetate (2 X 50 ml), the combined organic layers were dried over MgSO₄, filtered, and concentrated, and the residue was chromatographed on silica gel (20% ethyl acetate in hexane) to provide allyl alcohol **52** (2.84 g, 75%).

### J: (5Z)-(9S, 11R, 15S)-11,15-Bis(tetrahydropyran-2-yloxy)-15-cyclohexyl-9-hydroxy-2,3,4,16,17,18,19,20-octanor-5-prostenol (53):

To a solution of **52** (1.7 g, 2.9 mmol) in 20 ml of THF at 0°C was added a 1 *M* solution of TBAF in THF (4.6 ml, 4.6 mmol). After 30 min 30 ml of saturated NH₄Cl was added, the mixture was extracted with ethyl acetate (3 X 30 ml), the combined organic layers were dried over MgSO₄, filtered, and concentrated, and the residue was chromatographed on silica gel (3/2 ethyl acetate/hexane) to yield diol **53** (1.29 g, 93%).

### K: (5Z)-(9S, 11R, 15S)-11,15-Bis(tetrahydropyran-2-yloxy)-15-cyclohexyl-9-hydroxy-3-oxa-16,17,18,19,20-pentanor-5-prostenoic acid t-butyl ester (54):

A mixture of **53** (1.29 g, 2.68 mmol), toluene (20 ml), 25% w/w aqueous NaOH (20 ml), Bu₄NHSO₄ (175 mg, 0.52 mmol), and *t*-butyl bromoacetate (2.6 g, 13.5 mmol) was vigorously stirred for 10 min at 0°C and 45 min at room temperature. The layers were separated, the aqueous layer was extracted with ethyl acetate (2 X 20 ml), the combined organic layers were dried over MgSO₄, filtered, and concentrated, and the residue was chromatographed on silica gel (40% ethyl acetate in hexane) to afford 54 (1.56 g, 98%).

### L: (5Z)-(9S, 11R, 15S)-11,15-Bis(tetrahydropyran-2-yloxy)-15-cyclohexyl-9-hydroxy-3- oxa-16,17,18,19,20-pentanor-5-prostenoic acid (55):

A mixture of **54** (2.0 g, 3.4 mmol), lithium hydroxide monohydrate (830 mg, 20 mmol), methanol (20 ml), and water (1 ml) was stirred for 1.5 h, 35 ml of saturated KH₂PO₄ was added to adjust the pH to ca. 6, and the mixture was extracted with ethyl acetate (3 X 25 m l). The combined organic layers were dried over Na₂SO₄, filtered, and concentrated to provide crude acid 55, which was used in the next step without purification.

### M: (5Z)-(9S, 11R, 15S)-11,15-Bis(tetrahydropyran-2-yloxy)-15-cyclohexyl-9-hydroxy-3-oxa-16,17,18,19,20-pentanor-5-prostenoic acid isopropyl ester (56):

A mixture of **55** from above, acetone (34 mL), isopropyl iodide (2.9 g, 17 mmol), and DBU (3.0 g, 20 mmol) was stirred overnight. The reaction was added to 40 ml of saturated NH₄Cl, the mixture was extracted with ethyl acetate (3 X 25 ml), the combined organic layers were dried over MgSO₄, filtered, and concentrated, and the residue was chromatographed on silica gel (1/1 ethyl acetate/hexane) to yield isopropyl ester **56** (1.26 g, 64% from *t*-butyl ester **58**).

### N: (5Z)-(9R, 11R, 15S)-11,15-Bis(tetrahydropyran-2-yloxy)-9-chloro-15-cyclohexyl-3-oxa-16,17,18,19,20-pentanor-5-prostenoic acid isopropyl ester (57):

To a solution of **56** (1.26 g, 2.2 mmol) in 13 ml of pyridine at 0°C was added dropwise methanesulfonyl chloride (600 mg, 5.2 mmol). After 2 h, the mixture was added to a suspension of Bu₄NCl (6.1 g, 21.9 mmol) in toluene (9 ml) and heated at 55-63°C for 4 h. After the reaction cooled to room temperature, 30 mL of saturated NH₄Cl was added, the mixture was extracted with ethyl acetate (3 X 30 ml), the combined organic layers were dried over MgSO₄, filtered, and concentrated, and the residue was chromatographed on silica gel (20% ethyl acetate in hexane) to provide chlorinated product **57** as a mixture with the corresponding 8,9-olefin (1.07 g, 81% calculated as the chloride). The 8,9-olefin by-product was separated in the next step.

### O: (5Z)-(9R, 11R, 15S)-9-Chloro-15-cyclohexyl-11,15-dihydroxy-16,17,18,19,20-pentanor-5-prostenoic acid isopropyl ester (XIV):

The mixture from above containing **57** and the 8,9-olefin (1.07 g, 1.79 mmol, calculated as the chloride) was added to a solution of isopropanol (14 m 1), water (1 ml), and 12 *M* HCl (2 ml), and was stirred for 2 h. The reaction was added to 50 ml of saturated NaHCO₃, the mixture was extracted with ethyl acetate (3 X 30 ml), the combined organic layers were dried over MgSO₄, filtered, and , concentrated, and the residue was purified by radial chromatography (5% isopropanol in toluene) to afford pure **XIV** (172 mg, 22%), as well as a mixture of containing mostly **XIV** and a smaller proportion (ca. 30%) of the corresponding 8,9-olefin (333 mg, 42% calculated as the chloride). ¹³C NMR (CDCl₃) δ 170.21 (C), 131.12 (CH), 127.19 (CH), 76.75 (CH), 75.44 (CH), 68.70 (CH), 67.67 (CH₂), 66.78 (CH₂), 61.02 (CH), 54.28 (CH), 51.18 (CH), 44.31 (CH₂), 44.19 (CH), 31.35 (CH₂), 31.19 (CH₂), 29.73 (CH₂), 29.17 (CH₂), 27.60 (CH₂) 26.50 (CH₂), 26.31 (CH₂), 26.16 (CH₂), 21.80 (CH₃). CI MS, *m/z* calcd. for C₂₃H₄₀O₅Cl (MH⁺) 431, found 431.

### EXAMPLE 11: SYNTHESIS OF COMPOUND XV

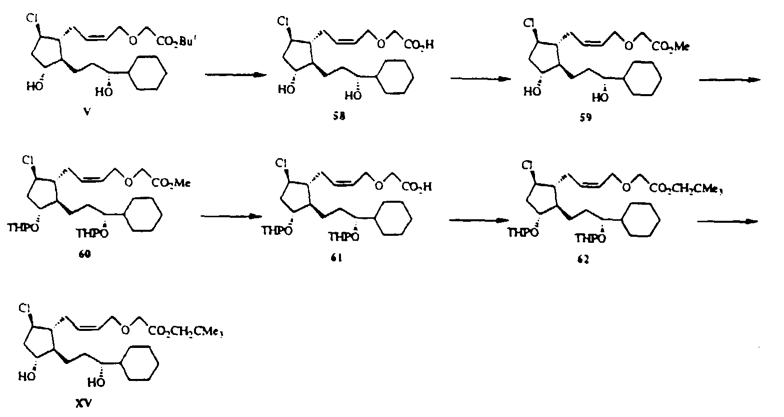

### A: (5Z)-(9R, 11R, 15R)-9-Chloro-15-cyclohexyl-11,15-dihydroxy-3-oxa-16,17,18,19,20-pentanor-5-prostenoic acid (58):

To a solution of 32 mg (0.072 mmol) of **V** (Example 1) in a mixture of 2.5 ml of methanol and 1.2 ml of water was added 15 mg (0.36 mmol) of lithium hydroxide monohydrate. After 9 h, 5 ml of 0.1 *M* HCl was added, and the mixture was extracted with CHCl₃ (5 X 5 m l). The combined organic layers were washed with water (20 ml) and brine (20 ml), dried over Na₂SO₄, filtered, and concentrated to afford 30 mg (100%) of 58 as a colorless oil. ¹³C NMR (CDCl₃) δ 173.17 (C), 132.77 (CH), 126.03 (CH), 75.68 (CH), 75.24 (CH), 66.89 (CH₂), 66.40 (CH₂), 61.32 (CH), 54.12 (CH), 50.62 (CH), 43.59 (CH₂), 43.33 (CH), 30.92 (CH₂), 30.73 (CH₂), 29.89 (CH₂), 29.30 (CH₂), 27.95 (CH₂), 26.44 (CH₂), 26.25 (CH₂), 26.09 (CH₂).

### B: (5Z)-(9R, 11R, 15R)-9-Chloro-15-cyclohexyl-11,15-dihydroxy-3-oxa-16,17,18,19,20-pentanor-5-prostenoic acid methyl ester (59):

To a solution of **58** (22 mg, 0.057 mmol) in 20 ml of ether was added excess diazomethane as a solution in ether. After 20 min residual diazomethane was removed from solution by a stream of N₂ and the solution was concentrated. The residue was chromatographed on silica gel (40% ethyl acetate in hexane) to afford **59** (21 mg, 91%). ¹³C NMR (CDCl₃) δ 171.06 (C), 131.75 (CH), 126.74 (CH), 75.73 (CH), 75.47 (CH), 67.34 (CH₂), 66.77 (CH₂), 61.08 (CH), 54.19 (CH), 51.94 (CH₃), 51.29 (CH), 44.47 (CH₂), 43.62 (CH), 31.49 (CH₂), 30.03 (CH₂), 29.30 (CH₂), 27.98 (CH₂), 26.49 (CH₂), 26.28 (CH₂), 26.13 (CH₂).

### C: (5Z)-(9R, 11R, 15R)-11,15-Bis(tetrahydropyran-2-yloxy)-9-chloro-15-cyclohexyl-3-oxa-16,17,18,19,20-pentanor-5-prostenoic acid methyl ester (60):

To a mixture of diol **59** (645 mg, 0.65 mmol), DHP (0.56 mL, 6.4 mmol), and CH₂Cl₂ (10 ml) at 0°C was added pTSA (10 mg, 0.053 mmol). After 15 min, 15 mL of saturated NaHCO₃ was added, the layers were separated, the aqueous layer was extracted with CH₂Cl₂ (3 X 15 ml), the combined organic layers were dried over MgSO₄, filtered, and concentrated, and the residue was chromatographed on silica gel (30% ethyl acetate in hexane) to afford 781 mg (86%) of bis-THP ether **60**.

### D: (5Z)-(9R, 11R, 15R)-11,15-Bis(tetrahydropyran-2-yloxy)-9-chloro-15-cyclohexyl-3-oxa-16,17,18,19,20-pentanor-5-prostenoic acid (61):

A solution of **60** (342 mg, 0.65 mmol) and lithium hydroxide monohydrate (96 mg, 2.3 mmol) in methanol (20 ml) was stirred for 2 h, the pH of the solution was adjusted to pH 6 with saturated KH₂PO₄, and the mixture was extracted with CH₂Cl₂ (6 X 20 ml). The combined organic layers were dried over Na₂SO₄, filtered, and concentrated to afford acid **61** (334 mg, 92%).

### E: (5Z)-(9R, 11R, 15R)-11,15-Bis(tetrahydropyran-2-yloxy)-9-chloro-15-cyclohexyl-3-oxa-16,17,18,19,20-pentanor-5-prostenoic acid neopentyl ester (62):

A mixture of **61** (80 mg, 0.14 mmol), dicyclohexylcarbodiimide (30 mg, 0.18 mmol), DMAP (8 mg, 0.07 mmol), 2,2-dimethyl-1-propanol (60 mg, 0.7 mmol), and CH₂Cl₂ (1 ml) was stirred for 3 h at room temperature, the solution was concentrated, and the residue was chromatographed on silica gel (20% ethyl acetate in hexane) to provide **62** contaminated with some dicyclohexyl urea (110 mg total). The sample was used in the next reaction without further purification.

### F: (5Z)-(9R, 11R, 15R)-9-Chloro-15-cyclohexyl-11,15-dihydroxy-3-oxa-16,17,18,19,20-pentanor-5-prostenoic acid neopentyl ester (XV):

The 110 mg sample from above was dissolved in a mixture of methanol (10 ml) and water (1 ml) at 0°C, and 10 drops of 12 *N* HCl was added. After 15 min, the solution was warmed to room temperature for 30 min. The solution was added to CH₂Cl₂ (10 ml), the layers were separated, and the aqueous layer was extracted with CH₂Cl₂ (3 X 10 ml). The combined organic layers were dried over MgSO₄, filtered, concentrated, and chromatographed on silica gel (1/1 ethyl acetate/hexane) to afford **XV** (46 mg, 71% from **61**). ¹³C NMR (CDCl₃) δ 170.88 (C), 131.82 (CH), 126.72 (CH), 75.68 (CH), 75.23 (CH), 74.20 (CH₂), 67.34 (CH₂) 66.76 (CH₂), 61.23 (CH), 54.24 (CH), 51.19 (CH), 44.43 (CH₂), 43.65 (CH), 31.44 (CH₂), 31.34 (C), 30.23 (CH₂), 30.09 (CH₂), 29.33 (CH₂), 28.00 (CH₂), 26.50 (CH₂), 26.37 (CH₃), 26.28 (CH₂), 26.14 (CH₂). CI MS *m/z* calcd. for C₂₅H₄₄O₅Cl (MH⁺) 459.2877, found 459.2872.

### EXAMPLE 12: SYNTHESIS OF COMPOUND XVI

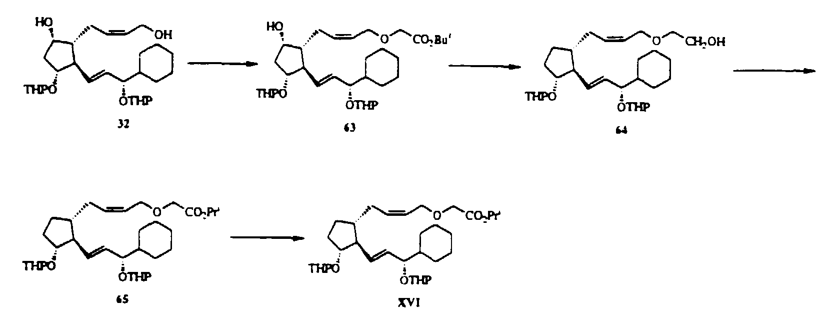

### A: (5Z, 13E)-(9S, 11R, 15S)-11,15-Bis(tetrahydropyran-2-yloxy)-15-cyclohexyl-9-hydroxy-3-oxa-16,17,18,19,20-pentanor-5,13-prostadienoic acid t-butyl ester (63)

To a vigorously stirring mixture of diol **32** (see Example 6) (925 mg, 1.93 mmol), toluene (25 ml), BrCH₂CO₂Bu^{*t*} (1.2 g, 6.3 mmol), and Bu₄HSO₄ (200 mg, 0.59 mmol) was added 20 ml of a 20% w/w aqueous solution of NaOH. After 40 min the layers were separated, the aqueous layer was extracted with ethyl acetate (2 X 30 ml), the combined organic layers were dried over MgSO₄, filtered, and concentrated, and the residue was chromatographed on silica gel eluting with 40% ethyl acetate in hexanes to afford **63** (1.10 g, 96%, R_{ƒ}= 0.5).

### B: (5Z, 13E)-(11R, 15S)-11,15-Bis(tetrahydropyran-2-yloxy)-15-cyclohexyl-3-oxa-16,17,18,19,20-pentanor-5,13-prostadienol (64)

To a mixture of **63** (1.09 g, 1.84 mmol) and pyridine (11 ml) at 0 °C was added dropwise methanesulfonyl chloride (0.51 g, 4.5 mmol). The reaction was stirred at 0 °C for 30 min and for 2 h at room temperature. 40 ml of saturated NH₄Cl was added, and the mixture was extracted with ethyl acetate (2 X 40 ml). The combined organic layers were dried over MgSO₄, filtered, and concentrated, and the residue was chromatographed on silica gel eluting with 1:1 hexane:ethyl acetate to afford the intermediate 9α-mesylate (1.08 g, 87%, R_{ƒ}= 0.5).

To a mixture of the mesylate in THF (11 ml) at 0 °C was added dropwise 1 *M* LiEt₃BH in THF (11 ml, 11 mmol) and the reaction was stirred overnight at room temperature. The mixture was poured into 50 mL of a 1:1 mixture of saturated NH₄Cl:ethyl acetate, the layers were separated, and the aqueous layer was extracted with ethyl acetate (2 X 30 ml). The combined organic layers were dried over MgSO₄, filtered, and concentrated, and the residue was chromatographed on silica gel eluting with 40% ethyl acetate in hexane to afford **64** (642 mg, 79%, R_{ƒ} = 0.4).

### C: (5Z, 12E)-(11R, 15S)-11,15-Bis(tetrahydropyran-2-yloxy)-15-cyclohexyl-3-oxa-16,17,18,19,20-pentanor-5,13-prostadienoic acid isopropyl ester (65)

To a mixture of **64** (560 mg, 1.12 mmol) and DMF (5 ml) was added pyridinium dichromate (1.32 g, 3.51 mmol). After stirring for 48 h. 20 ml of water was added, and the mixture was extracted with ethyl acetate (4 X 20 ml). The combined organic layers were filtered and concentrated. the residue was dissolved in acetone (20 ml), and DBU was added (780 mg, 5.12 mmol), followed in 15 min by the addition of isopropyl iodide (850 mg, 5.0 mmol). After 20 h, the reaction was concentrated, and the residue was
chromatographed on silica gel eluting with 20% ethyl acetate in hexane to afford **65** (238 mg, 38%, R_{ƒ}= 0.4).

### D: (5Z, 13E)-(11R, 15S)-15-Cyclohexyl-11,15-dihydroxy-3-oxa-16,17,18,19,20-pentanor-5,13-prostadienoic acid isopropyl ester (XVI)

To a mixture of **65** (230 mg, 0.41 mmol), isopropanol (18 ml), and water (2 ml) was added 12 *M* HCl (1 ml). After 2 h, 20 ml of saturated NaHCO₃ was added, the mixture was extracted with ethyl acetate (3 X 20 ml), the combined organic layers were dried over MgSO₄, filtered, and concentrated, and the residue was chromatographed on silica gel eluting with 3:2 ethyl acetate:hexane to afford **XVI** (120 mg, 74%, R_{ƒ} = 0.25). ¹³C NMR (CDCl₃) δ 170.00 (C), 134.17 (CH), 132.73 (CH), 125.97 (CH), 77.92 (CH), 77.68 (CH), 68.47 (CH), 67.38 (CH₂), 66.73 (CH₂), 58.02 (CH), 43.44 (CH), 42.77 (CH), 32.15 (CH₂), 28.89 (CH2), 28.80 (CH2), 27.63 (CH₂), 26.51 (CH2), 26.06 (CH2). 25.94 (CH₂), 21.97 (CH₃).

The compounds of formulae (I) and (II) are useful in lowering intraocular pressure and thus are useful in the treatment of glaucoma. The preferred route of administration is topical. The dosage range for topical administration is generally between about 0.001 and about 1000 micrograms per eye (µg/eye) and is preferably between about 0.01 and about 100 µg/eye and most preferably between about 0.05 and 50 µg/eye. The compounds of the present invention can be administered as solutions, suspensions, or emulsions (dispersions) in a suitable ophthalmic vehicle.

In forming compositions for topical administration, the compounds of the present invention are generally formulated as between about 0.00002 to about 0.5 percent by weight (wt%) solutions in water at a pH between about 4.5 and about 8.0. The compounds are preferably formulated as between about 0.0001 to about 0.1 wt% and, most preferably, between about 0.001 and about 0.05 wt%. While the precise regimen is left to the discretion of the clinician, it is recommended that the compositions be topically applied by placing one or more drops in each eye one or more times a day.

Other ingredients which may be desirable to use in the ophthalmic preparations of the present invention include preservatives, co-solvents and viscosity building agents.

### Antimicrobial Preservatives:

Ophthalmic products are typically packaged in multidose form, which generally require the addition of preservatives to prevent microbial contamination during use. Suitable preservatives include: benzalkonium chloride, thimerosal, chlorobutanol, methyl paraben, propyl paraben, phenylethyl alcohol, edetate disodium, sorbic acid, ONAMER M®, or other agents known to those skilled in the art. Such preservatives are typically employed at a concentration between about 0.001 and about 1.0 wt%.

### Co-Solvents:

Prostaglandins, and particularly ester derivatives, typically have limited solubility in water and therefore may require a surfactant or other appropriate cosolvent in the composition. Such co-solvents include: Polysorbate 20. 60 and 80; Pluronic F-68, F-84 and P-103 (trademarks); Tyloxapol (trademark); Cremophor EL (trademark); sodium dodecyl sulfate; glycerol; PEG 400; propylene glycol; cyclodextrins; or other agents known to those skilled in the art. Such co-solvents are typically employed at a concentration between about 0.01 and about 2 wt%.

### Viscosity Agents:

Viscosity greater than that of simple aqueous solutions may be desirable to increase ocular absorption of the active compound, to decrease variability in dispensing the formulations, to decrease physical separation of components of a suspension or emulsion of formulation and/or otherwise to improve the ophthalmic formulation. Such viscosity building agents include: polyvinyl alcohol; polyvinyl pyrrolidone; cellulosic polymers, such as methyl cellulose, hydroxy propyl methylcellulose, hydroxyethyl cellulose, carboxymethyl cellulose, hydroxy propyl cellulose; carboxy vinyl polymers, such as carbomer 910, carbomer 940, carbomer 934P and carbomer 1342; or other agents known to those skilled in the art. Such agents are typically used at a concentration between about 0.01 and about 2 wt%.

### EXAMPLE 13

The following Tables 2-4 containing Formulations A-M are representive pharmaceutical compositions of the Invention for topical use in lowering of intraocular pressure. Each of Formulations A-M may be formulated in accordance with procedures known to those skilled in the art.

**TABLE 2**

| **INGREDIENT** | **FORMULATION (wt%)** | | | |
|---|---|---|---|---|
| | **A** | **B** | **C** | **D** |
| Compound **III** | --- | 0.01 | --- | --- |
| Compound **IV** | 0.002 | --- | --- | --- |
| Compound **XVI** | --- | --- | 0.05 | --- |
| Compound **XVII** | --- | --- | --- | 0.1 |
| Dextran 70 | 0.1 | 0.1 | --- | --- |
| Hydroxypropyl Methylcellulose | 0.3 | 0.5 | --- | --- |
| Monobasic Sodium Phosphate | --- | 0.05 | 0.05 | 0.05 |
| Dibasic Sodium Phosphate (anhydrous) | -- | 0.15 | 0.15 | 0.15 |
| Sodium Chloride | 0.77 | 0.75 | 0.5 | 0.6 |
| Potassium Chloride | 0.12 | --- | --- | --- |
| Disodium EDTA | 0.05 | 0.05 | --- | --- |
| Hiydroxypropyl-β-cyclodextrin | --- | --- | 1.0 | --- |
| Tyloxapol | --- | 0.1 | --- | 0.4 |
| Benzalkonium Chloride | 0.01 | 0.01 | 0.01 | 0.02 |
| Polysorbate 80 | 0.01 | --- | --- | --- |
| HCl and/or NaOH | q.s. to pH 7.2-7.5 | q.s. to pH 7.3-7.4 | q.s. to pH 6.3-6.6 | q.s. to pH 6.3-6.6 |
| Purified Water | q.s. to 100% | q.s. to 100% | q.s. to 100% | q.s. to 100% |

**TABLE 3**

| **INGREDIENT** | **FORMULATION (wt%)** | | | |
|---|---|---|---|---|
| | **E** | **F** | **G** | **H** |
| Compound **V** | 0.01 | --- | --- | --- |
| Compound **VI** | --- | 0.01 | --- | --- |
| Compound **XVII** | --- | --- | 0.1 | --- |
| Compound **XIX** | --- | - | --- | 0.2 |
| Monobasic Sodium Phosphate | 0.05 | 0.05 | 0.05 | 0.05 |
| Dibasic Sodium Phosphate (anhydrous) | 0.15 | 0.15 | 0.15 | 0.15 |
| Sodium Chloride | 0.75 | 0.75 | 0.5 | 0.6 |
| Disodium EDTA | 0.01 | 0.05 | --- | --- |
| Cremophor® EL | -- | 0.1 | --- | --- |
| Hydroxypropyl-β-cyclodextrin | --- | --- | 4.0 | --- |
| Tyloxapol | --- | --- | --- | 0.5 |
| Benzalkonium Chloride | 0.02 | 0.01 | 0.01 | 0.02 |
| Polysorbate 80 | 0.05 | --- | --- | --- |
| HCl and/or NaOH | q.s. to pH 7.3-7.4 | q.s. to pH 7.3-7.4 | q.s. to pH 6.3-6.6 | q.s. to pH 6.3-6.6 |
| Purified Water | q.s. to 100% | q.s. to 100% | q.s. to 100% | q.s. to 100% |

**TABLE 4**

| **INGREDIENT** | **FORMULATION (wt%)** | | | |
|---|---|---|---|---|
| | **J** | **K** | **L** | **M** |
| Compound **VIII** | 0.01 | --- | --- | --- |
| Compound **IX** | --- | 0.01 | --- | --- |
| Compound **X** | --- | -- | 0.1 | 0.2 |
| Monobasic Sodium Phosphate | 0.05 | 0.05 | 0.05 | 0.05 |
| Dibasic Sodium Phosphate (anhydrous) | 0.15 | 0.15 | 0.15 | 0.15 |
| Sodium Chloride | 0.75 | 0.75 | 0.5 | 0.6 |
| Disodium EDTA | 0.01 | 0.05 | --- | --- |
| Cremophor® EL | --- | 0.01 | --- | --- |
| Hydroxypropyl-β-cyclodextrin | --- | --- | 4.0 | --- |
| Tyloxapol | --- | --- | --- | 0.5 |
| Benzalkonium Chloride | 0.02 | 0.01 | 0.01 | 0.02 |
| Polysorbate 80 | 0.05 | --- | --- | --- |
| HCl and/or NaOH | q.s. to pH 7.3-7.4 | q.s. to pH 7.3-7.4 | q.s. to pH 6.3-6.6 | q.s. to pH 6.3-6.6 |
| Purified Water | q.s. to 100% | q.s. to 100% | q.s. to 100% | q.s. to 100% |

### EXAMPLE 14

The ability of certain compounds of the present invention to reduce intraocular pressure (IOP) was evaluated in cynomolgus monkeys with ocular hypertension produced by previous laser trabeculoplasty in the right eye. Animals had been trained to sit in restraint chairs and conditioned to accept experimental procedures without chemical restraint. IOP was determined with a pneumatonometer after light corneal anesthesia with dilute proparacaine. The test protocol included a treatment regimen consisting of 5 divided doses administered over a period of 2 and 1/2 days. Doses 2-5 were given 8, 24, 32 and 48 hours after the initial dose. Baseline IOP values were determined prior to treatment with the test formulation, and then IOP was determined 16 hours after the fourth dose, and 2, 4 and 6 hours after the fifth dose. Prostaglandin doses are micrograms of compound contained in each treatment.

The two tested compounds are the previously identified Compounds **III-VII**.

**TABLE 5**

| **Compound** | **PG PG Dose** | **Baseline IOP (mm Hg)** | **Percent IOP Reduction at Hours after Dose/Dose#** | | | |
|---|---|---|---|---|---|---|
| | | | **16/4** | **2/5** | **4/5** | **6/5** |
| **III** | 1 µg | 31.1 | 15.6±4.7 | 33.7±5.5 | 28.1±4.1 | 31.0±4.6 |
| **IV** | 1 µg | 31.3 | 47.6±2.6 | 52.8±3.3 | 54.7±3.8 | 53.1±4.1 |
| **V** | 5 µg | 36.3 | 38.3±3.0 | 47.5±5.1 | 43.7±5.6 | 36.5±5.8 |
| **VI** | 1 µg | 36.9 | 22.3±2.7 | 30.8±4.7 | 26.4±4.7 | 24.9±4.0 |
| **VII** | 20 µg | 32.6 | 13.9±2.7 | 30.1±4.8 | 22.4±5.2 | 19.3±4.3 |

Results are presented in Table 5, above. Compounds **III-VII** produced significant reduction of intraocular pressure at doses which are marginal or ineffective for other prostaglandins in published clinical studies. Compound **IV** was especially potent, producing greater than 50% reduction of intraocular pressure with just 1 µg of compound. In contrast, Nakajima et al. (Graefe's Arch. Clin. Exp. Ophthalmol., 229:411-413 (1991)) reported that 50 µg of PGD₂ and 2.5 µg of BW245C (a PGD₂ analogue) reduced intraocular pressure in human eyes by 12% and 10%, respectively. Other studies (Woodward et al., Invest. Ophthalmol. Vis. Sci., 31:138-146 (1990)) reported for these reference compounds in rabbits describe a maximum IOP reduction of approximately 28% for 250 µg of PGD₂ and 22% for 25 µg of BW245C. These comparisons indicate the unexpected potency of Compounds **III - VII** in reducing intraocular pressure. No indications of inflammation were observed during these studies.

### EXAMPLE 15

The ability of certain compounds of the present invention to reduce intraocular pressure (IOP) was evaluated in cynomolgus monkeys with ocular hypertension produced by previous laser trabeculoplasty in the right eye. Animals had been trained to sit in restraint chairs and conditioned to accept experimental procedures without chemical restraint. IOP was determined with a pneumatonometer after light corneal anesthesia with dilute proparacaine.

The two compounds tested are those previously identified as Compound **XI** and Compound **XII**.

**TABLE 7**

| **COMPOUND** | **PG DOSE** | **MAXIMUM PERCENT IOP REDUCTION FROM BASELINE** |
|---|---|---|
| **XI** | 3 µg | 42 |
| **XII** | 0.3 µg | 44 |

Compounds **XI** and **XII** produced significant reduction of intraocular pressure at doses which are marginal or ineffective for other prostaglandins in published clinical studies. By comparison, Nakajima et al. (Graefe's Arch. Clin. Exp. Ophthalmol. 229:411-413 (1991)) reported that 50 µg of PGD₂ and 2.5 µg of BW245C (a PGD₂ analogue) reduce intraocular pressure in human eyes by 12% and 10%, respectively. Other studies (Woodward et al., Invest. Ophthalmol. Vis. Sci. 31:138-146 (1990)) reported for these reference compounds in rabbits describe a maximum IOP reduction of approximately 28% for 250 µg of PGD₂ and 22% for 25 µg of BW245C. These comparisons indicate the unexpected potency of compounds of the present invention in reducing intraocular pressure. No indications of inflammation were observed during these studies.

## Claims

1. A topical ophthalmic composition for the treatment of glaucoma and ocular hypertension, said composition comprising an ophthalmically acceptable vehicle and a therapeutically effective amount of a compound of formula: 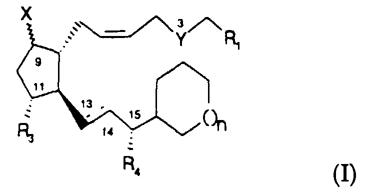 wherein:
R₁ = CO₂R₂, wherein R₂ = H, a cationic salt moiety, or an ophthalmically acceptable ammonium moiety; or R₁ may also represent an ophthalmically acceptable ester moiety;
X = halogen, particularly Cl or F, in either configuration;
Y = CH₂ or O;
--- = single or *trans* double bond;
R₃, R₄ can be the same or different, and are selected from: free or functionally modified hydroxy groups;
n = 0 or 1; and
() represents the fact that the ring may be 5-membered or 6-membered.

2. The composition of claim 1, wherein: R₁ = CO₂R₂; R₂ = substituted or unsubstituted alkyl, cycloalkyl, (cycloalkyl)alkyl, aryl, arylalkyl, heteroaryl, or (heteroaryl)alkyl, wherein substituents are selected from the group consisting of: alkyl, halogen, a free or functionally modified hydroxy group, or a free or functionally modified thiol; and X = F or Cl in either configuration.

3. The composition of claim 2, wherein: R₁ = CO₂R₂; R₂ = H, methyl, ethyl, n-propyl, isopropyl, t-butyl or benzyl; X = Cl in the β (*R*) configuration; Y = O; R₃ and R₄ = OH; and n = 1.

4. The composition of claim 3, wherein: R₂ = t-butyl.

5. The composition of claim 2, wherein: R₁ = CO₂R₂; R₂ = H, methyl, ethyl, n-propyl, isopropyl, t-butyl, or benzyl; X = Cl in the β (*R*) configuration; Y = CH₂; R₃ and R₄ = OH; and n = 1.

6. The composition of claim 5, wherein: R₂ = methyl.

7. The composition of any of claims 1-5 or 28, wherein the compound of formula (I) is present at a concentration between about 0.00002 and about 0.5 percent by weight.

8. The composition of claim 7, wherein the compound of formula (I) is present at a concentration between about 0.0001 and about 0.1 percent by weight.

9. The composition of claim 8, wherein the compound of formula (I) is present at a concentration between about 0.001 and about 0.05 percent by weight.

10. Use of the composition of any of claims 1-5 or 28 for the manufacture of a medicament for the topical administration of said composition to a glaucomatous or hypertensive eye.

11. Use according to claim 10, wherein between about 0.001 and about 1000 micrograms of a compound of formula (I) is administered.

12. Use according to claim 11, wherein between about 0.01 and about 100 micrograms of a compound of formula (I) is administered.

13. Use according to claim 12, wherein between about 0.05 and about 50 micrograms of a compound of formula (I) is administered.

14. A topical ophthalmic composition for the treatment of glaucoma and ocular hypertension, said composition comprising an ophthalmically acceptable vehicle and a therapeutically effective amount of a compound of formula: 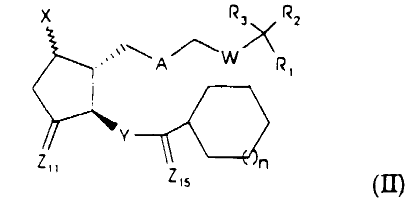 wherein:
R₁ = CH₂R, CO₂R₄;
R = OH or functionally modified hydroxy group;
R₂ and R₃ can be the same or different and are selected from: H and CH₃;
R₄ = H, a cationic salt moiety, substituted or unsubstituted alkyl, cycloalkyl, (cydoalkyl)alkyl, aryl, arylalkyl, heteroaryl, or (heteroaryl)alkyl, wherein substituents include alkyl, halo, a free or functionally modified hydroxy group or a free or functionally modified thiol;
W = CH₂, O, S(O)ₘ wherein m = 0, 1, 2;
A = CH₂CH₂, *cis* or *trans* CH=CH, or C≡C;
X = Cl, F or R in either configuration, or H;
Z₁₁ and Z₁₅ may be the same or different and may be selected from O, H and R in either configuration, or H and H;
Y = CH₂CH₂ or *trans* CH=CH, or C≡C;
n = 0 or 1; and
() represents the fact that the ring may be 5-membered or 6-membered.
with the proviso that the following compounds be excluded: 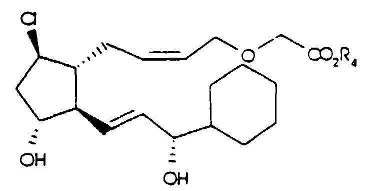 wherein R₄ is as defined above.

15. The composition of claim 14, wherein: W = CH₂ or O; A = *cis* CH=CH; X = Cl or H; Z₁₁ = R and H in either configuration, or H and H; Y = CH₂CH₂, or *trans* CH=CH; and n = 1.

16. The composition of claim 15, wherein: R₂, R₃ = H; X = Cl in β-configuration, or H; Z₁₅ = H and R in either configuration; and R₄=H, a cationic salt moiety, or substituted or unsubstituted C₁-C₁₀ alkyl.

17. The composition of any of claims 14-16, wherein the compound of formula (II) is present at a concentration between about 0.00002 and about 0.5 percent by weight.

18. The composition of claim 17, wherein the compound of formula (II) is present at a concentration between about 0.0001 and about 0.1 percent by weight.

19. The composition of claim 18, wherein the compound of formula (II) is present at a concentration between about 0.001 and about 0.05 percent by weight.

20. Use of the composition of any of claims 14-16 for the manufacture of a medicament for the topical administration of said composition to a glaucomatous or hypertensive eye.

21. Use according to claim 20, wherein between about 0.001 and about 1000 micrograms of a compound of formula (II) is administered.

22. Use according to claim 21, wherein between about 0.01 and about 100 micrograms of a compound of formula (II) is administered.

23. Use according to claim 22, wherein between about 0.05 and about 50 micrograms of a compound of formula (II) is administered.

24. A compound of formula: 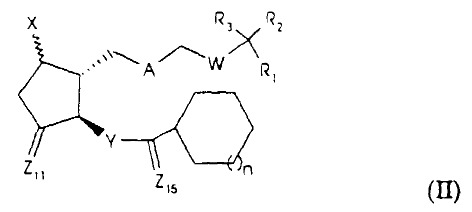 wherein:
R₁ = CH₂R, CO₂R₄;
R = OH or functionally modified (i.e., etherified and acylated) hydroxy group;
R₂ and R₃ can be the same or different and are selected from: H and CH₃;
R₄ = H, a cationic salt moiety, substituted or unsubstituted alkyl, cycloalkyl, (cycloalkyl)alkyl, aryl, arylalkyl, heteroaryl, or (heteroaryl)alkyl, wherein substituents indude alkyl, halo, a free or functionally modified hydroxy group or a free or functionally modified thiol;
W = O, S(O)ₘ wherein m = 0, 1, 2;
A = CH₂CH₂, *cis* or *trans* CH=CH, or C≡C;
X = H;
Z₁₁ and Z₁₅ may be the same or different and may be selected from O, H and R in either configuration or H and H;
Y = CH₂CH₂ or *trans* CH=CH, or C≡C;
n = 0 or 1; and
() represents the fact that the ring may be 5-membered or 6-membered.

25. A compound of claim 24 wherein: R₁ = CO₂R₄ R₂ and R₃ = H; R₄=H, a cationic salt moiety, or a substituted or unsubstituted C₁-C₁₀ alkyl; W = O, A = *cis* CH=CH; Z₁₁ and Z₁₅ = R and H in either configuration; Y = CH₂CH₂, or *trans* CH=CH; and n = 0 or 1.

26. A compound of claim 25, having the formula:

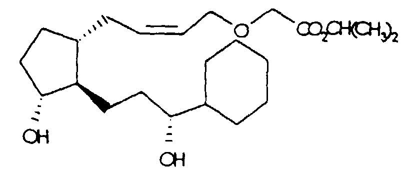

27. A compound of claim 25, having the formula:

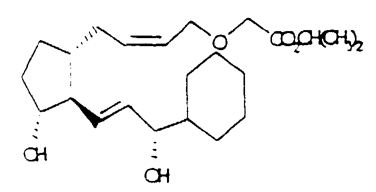

28. The composition of claim 3, wherein R₁ = CO₂R₂ and R₂ = isopropyl.
